(19) **Europäisches Patentamt / European Patent Office / Office européen des brevets**

(11) **EP 4 759 829 A1**

(12) **EUROPEAN PATENT APPLICATION**
published in accordance with Art. 153(4) EPC

(43) Date of publication:
**17.06.2026 Bulletin 2026/25**

(21) Application number: **24851970.4**

(22) Date of filing: **09.08.2024**

(51) International Patent Classification (IPC):
**C07K 16/28** (2006.01)  **A61K 39/395** (2006.01)
**A61K 45/00** (2006.01)  **A61K 47/68** (2017.01)
**A61P 35/00** (2006.01)  **A61P 43/00** (2006.01)
**C07K 16/46** (2006.01)  **C12N 15/13** (2006.01)
**C12N 15/63** (2006.01)

(52) Cooperative Patent Classification (CPC):
**A61K 39/395; A61K 45/00; A61K 47/68;**
**A61P 35/00; A61P 43/00; C07K 16/00;**
**C07K 16/28; C07K 16/46; C12N 15/63**

(86) International application number:
**PCT/JP2024/028824**

(87) International publication number:
**WO 2025/033553 (13.02.2025 Gazette 2025/07)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.08.2023 JP 2023131057**

(71) Applicants:
• **The University of Tokyo**
  **Bunkyo-ku, Tokyo 113-8654 (JP)**
• **RIKEN**
  **Wako-shi, Saitama 351-0198 (JP)**

(72) Inventors:
• **MIYAZONO, Kohei**
  **Tokyo 113-8654 (JP)**

• **TANABE, Ryo**
  **Tokyo 113-8654 (JP)**
• **HELDIN, Carl-Henrik**
  **Tokyo 113-8654 (JP)**
• **WESTERMARK, Bengt Anders**
  **Tokyo 113-8654 (JP)**
• **MATSUMOTO, Takehisa**
  **Wako-shi, Saitama 351-0198 (JP)**
• **SHIROUZU, Mikako**
  **Wako-shi, Saitama 351-0198 (JP)**

(74) Representative: **Regimbeau**
  **20, rue de Chazelles**
  **75847 Paris Cedex 17 (FR)**

Remarks:
The complete document including Reference
Table(s) and the Sequence Listing(s) can be
downloaded from the EPO website

(54) **NOVEL HVEM-BINDING PROTEIN AND PHARMACEUTICAL COMPOSITION CONTAINING SAME**

(57) An object of the present invention is to provide a novel HVEM-binding protein. According to the present invention, there is provided a human HVEM-binding protein including: at least one immunoglobulin single variable region, in which the immunoglobulin single variable region includes complementarity determining regions 1 to 3 (CDR1, CDR2, and CDR3), each having an amino acid sequence represented by SEQ ID NO: 1, an amino acid sequence represented by SEQ ID NO: 2, and an amino acid sequence represented by SEQ ID NO: 3, respectively, and the immunoglobulin single variable region is a humanized VHH. The protein of the present invention may further include a human immunoglobulin Fc region or a human serum albumin-binding protein, and the immunoglobulin Fc region may be the human IgG4 Fc region.

**EP 4 759 829 A1**

**FIGURE 9**

**Description**

CROSS-REFERENCE TO RELATED APPLICATION

The present application enjoys the benefit of priority from the prior Japanese Patent Application No. 2023-131057 filed on August 10, 2023, the entire disclosures of which are incorporated herein by reference.

TECHNICAL FIELD

The present invention relates to an HVEM-binding protein and a pharmaceutical composition comprising the protein.

BACKGROUND ART

The incidence of primary brain tumors in Japan is estimated to be approximately 20,000 cases per year (with a prevalence of 130.8 per 100,000 people in fiscal year 2010), and the so-called five major brain tumors, that is, glioma, meningioma, pituitary adenoma, schwannoma, and craniopharyngioma are known. It has also been reported that, in Europe and the United States, the incidence of primary brain tumors is approximately twice as high as in Japan for the malignant tumor glioblastoma multiforme. Treatment of brain tumors generally involves surgical resection of the tumor. However, in the case of malignant brain tumors, multidisciplinary treatment combining anticancer agents and radiation therapy is further carried out. The therapeutic effect depends on how much of the tumor could be resected in the initial surgery, and in the case of malignant tumors, it has been reported that unless more than 95 to 98% of the entire tumor is resected, there is little difference in prognosis compared to cases where only half of the tumor was resected. It is also said that, among brain tumors, malignant brain tumors tend to progress to brain tumors with a higher degree of malignancy with high probability.

Glioblastoma multiforme (hereinafter may be referred to as "GBM") is the most common and most malignant form of malignant brain tumor in adults. Despite advances in surgery, radiotherapy, and chemotherapy, the prognosis of patients with glioblastoma still remains poor, with a median survival period of less than 15 months. Glioblastoma is classified into Proneural, Neural, Classical, and Mesenchymal subtypes based on genomic abnormalities according to the Cancer Genome Atlas (TCGA) dataset. The highly proliferative nature of glioblastoma cells is due to alterations in several signaling pathways, including oncogenes and tumor suppressor genes.

Bone morphogenetic proteins (hereinafter may be referred to as "BMP") are members of the TGF-$\beta$ superfamily, and members of the BMP subfamily including BMP-2, BMP-4, and BMP-7 have been reported to induce differentiation, cell cycle arrest, and apoptosis of glioblastoma cells. Thus far, BMP target genes identified in glioblastoma have included many intracellular signaling molecules (Non Patent Document 1). However, it has also been reported that the Mesenchymal subtype of glioblastoma often exhibits resistance to BMP (Non Patent Document 2).

Reference List

Non Patent Documents

Non Patent Document 1: Savary K et al., Oncogene, 32, 5409-5420 (2013)
Non Patent Document 2: Dalmo et al., Mol. Cancer Res., 18, 981-991 (2020)

SUMMARY OF THE INVENTION

Technical Problem

An object of the present invention is to provide a novel HVEM-binding protein and a pharmaceutical composition comprising the protein.

Solution to Problem

According to the present invention, the following invention is provided.

[1] A human HVEM-binding protein comprising: at least one immunoglobulin single variable region, in which

the immunoglobulin single variable region comprises complementarity determining regions 1 to 3 (CDR1, CDR2, and CDR3), each having an amino acid sequence represented by SEQ ID NO: 1, an amino acid sequence represented by SEQ ID NO: 2, and an amino acid sequence represented by SEQ ID NO: 3, respectively, and the immunoglobulin single variable region is a humanized VHH.

[2] The HVEM-binding protein according to the above [1], in which the immunoglobulin single variable region comprises an amino acid sequence of (v), (vi), or (vii) below,

(v) an amino acid sequence of SEQ ID NO: 18, 19, or 20,
(vi) an amino acid sequence having at least 80% identity to the sequence of the framework region (excluding amino acids corresponding to a humanized region) in the sequence of (v), and
(vii) an amino acid sequence in which one or more amino acids are deleted, substituted, inserted, and/or added in the sequence of the framework region (excluding amino acids corresponding to the humanized region) in the sequence described in (v) or (vi).

[3] The HVEM-binding protein according to the above [1] or [2], in which the immunoglobulin single variable region comprises framework regions (FR1, FR2, FR3, and FR4), and the amino acid sequences of FR1, FR2, FR3, and FR4 are (i), (ii), or (iii) below,

(i) FR1 having an amino acid sequence represented by SEQ ID NO: 5,

FR2 having an amino acid sequence represented by SEQ ID NO: 8,
FR3 having an amino acid sequence represented by SEQ ID NO: 11, and
FR4 having an amino acid sequence represented by SEQ ID NO: 13,

(ii) FR1 having an amino acid sequence represented by SEQ ID NO: 5,

FR2 having an amino acid sequence represented by SEQ ID NO: 7,
FR3 having an amino acid sequence represented by SEQ ID NO: 10, and
FR4 having an amino acid sequence represented by SEQ ID NO: 13, and

(iii) FR1 having an amino acid sequence represented by SEQ ID NO: 5,

FR2 having an amino acid sequence represented by SEQ ID NO: 6,
FR3 having an amino acid sequence represented by SEQ ID NO: 10, and
FR4 having an amino acid sequence represented by SEQ ID NO: 13.

[4] The HVEM-binding protein according to any one of the above [1] to [3], further comprising a human immunoglobulin Fc region or a human serum albumin-binding protein.

[5] The HVEM-binding protein according to the above [4], in which the immunoglobulin Fc region is a human IgG4 Fc region.

[6] The HVEM-binding protein according to the above [5], in which the amino acid sequence of the human IgG4 Fc region is an amino acid sequence of SEQ ID NO: 14, 15, or 16.

[7] An antibody-drug conjugate comprising the HVEM-binding protein according to the above [1] or [4] and a drug.

[8] The antibody-drug conjugate according to the above [7], in which the drug is an anticancer agent.

[9] A pharmaceutical composition comprising the HVEM-binding protein according to any one of the above [1] to [6], or the antibody-drug conjugate according to the above [7].

[10] The pharmaceutical composition according to the above [9] for use in inhibiting the binding of HVEM to BTLA and/or APRIL.

[11] The pharmaceutical composition according to the above [9] for use in the treatment or prevention of solid cancer or malignant tumor.

[12] The pharmaceutical composition according to any one of the above [9] to [11] for use in the treatment or prevention of gliomas.

[13] The pharmaceutical composition according to the above [12], in which the gliomas are any one of an oligodendroglioma, an oligoastrocytoma, an astrocytoma, or a glioblastoma multiforme.

[14] The pharmaceutical composition according to the above [13], in which the glioblastoma multiforme belongs to any one of the Proneural subtype, Neural subtype, Classical subtype, or Mesenchymal subtype.

[15] The pharmaceutical composition according to any one of the above [9] to [14] for use in combination with another anticancer agent.

[16] An agent for inhibiting an HVEM signaling pathway, comprising the HVEM-binding protein according to any one of the above [1] to [6].

[17] The agent according to the above [16], in which the HVEM signaling pathway is an NF-κB signaling pathway via HVEM.

[18] An agent for inducing anticancer immunity comprising the HVEM-binding protein according to any one of the above [1] to [6].

[19] A polynucleotide encoding the HVEM-binding protein according to any one of the above [1] to [6].

[20] An expression vector comprising the polynucleotide according to the above [19].

[21] A method for treating or preventing solid cancer, comprising administering to a subject in need thereof the HVEM-binding protein according to any one of the above [1] to [6] or a composition comprising the same, or the antibody-drug conjugate according to the above [7] or a composition comprising the same.

[22] Use of the HVEM-binding protein according to any one of the above [1] to [6], or the antibody-drug conjugate according to the above [7], for the production of an agent for treating or preventing solid cancer.

The protein of the above [1] may be referred to as the "protein of the present invention" in the present specification, and the composition of the above [9] may be referred to as the "composition of the present invention" in the present specification.

According to the present invention, the invention is advantageous in that an HVEM-binding protein having an activity of selectively inhibiting human HVEM and exhibiting good pharmacokinetics and a pharmaceutical composition comprising the protein can be provided.

BRIEF DESCRIPTION OF THE DRAWINGS

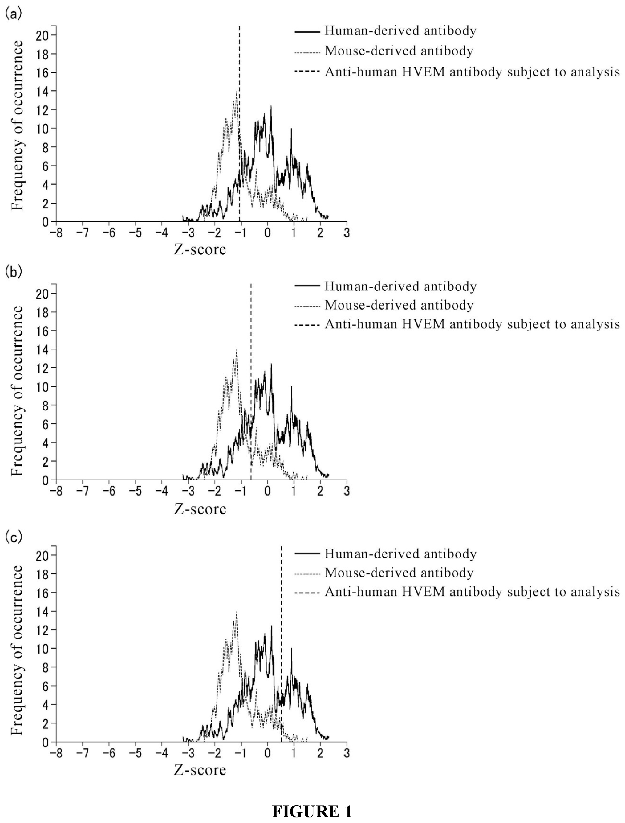

Fig. 1 is a diagram showing a degree of humanization of an alpaca anti-human HVEM antibody (VHH-3, SEQ ID NO: 17) (Fig. 1a), a single variable region (SEQ ID NO: 19) of a humanized anti-human HVEM antibody (HVs80) (Fig. 1b), and a single variable region (SEQ ID NO: 20) of humanized anti-human HVEM antibodies (HVR80 and HVR81) (Fig. 1c) (Example 3).

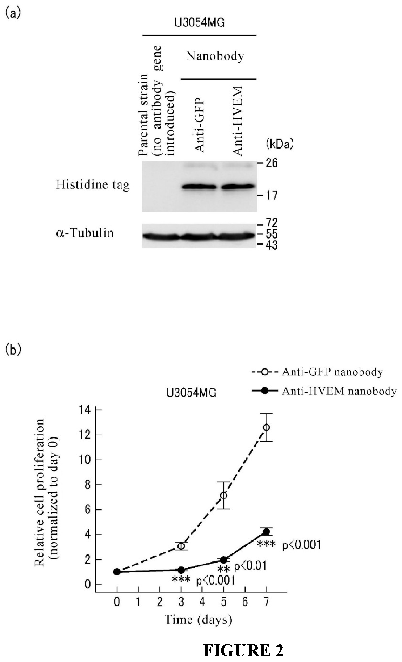

Fig. 2 is a diagram showing the effects of gene introduction of an alpaca anti-human HVEM antibody on proliferation of Mesenchymal subtype cells (Example 5). Fig. 2(a) is a diagram showing an expression of an antibody after introducing a gene of an alpaca anti-human HVEM antibody (VHH-3) or an anti-GFP antibody (negative control) into U3054MG of Mesenchymal subtype GBM cells. Fig. 2(b) is a diagram showing that the proliferation of Mesenchymal subtype GBM cells is suppressed by introducing the gene of the alpaca anti-human HVEM antibody (VHH-3) (biological replicates, n = 3).

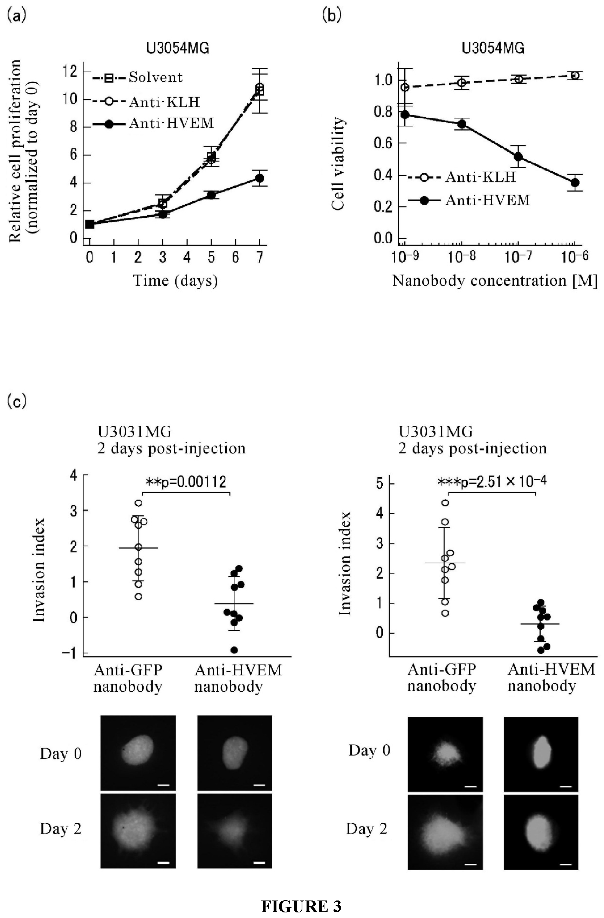

Fig. 3 is a diagram showing the effects of an alpaca anti-human HVEM antibody on proliferation and cell invasion of Mesenchymal subtype cells (Example 6). Figs. 3(a) and 3(b) show the effects of alpaca anti-human HVEM antibody (VHH-3) on proliferation of Mesenchymal subtype cells. Fig. 3(a) shows a proliferation curve, and Fig. 3(b) shows a dose-response curve (each with biological replicates, n = 2). Fig. 3(c) is a diagram showing the inhibitory effect of the alpaca anti-human HVEM antibody on the cell invasiveness by culturing spheroids of Mesenchymal subtype GBM cells (U3031MG or U3054MG) on mouse brain slices (biological replicates, n = 9). Representative images for each experimental condition (corresponding to the median of the cell invasion index) are shown below the graph.

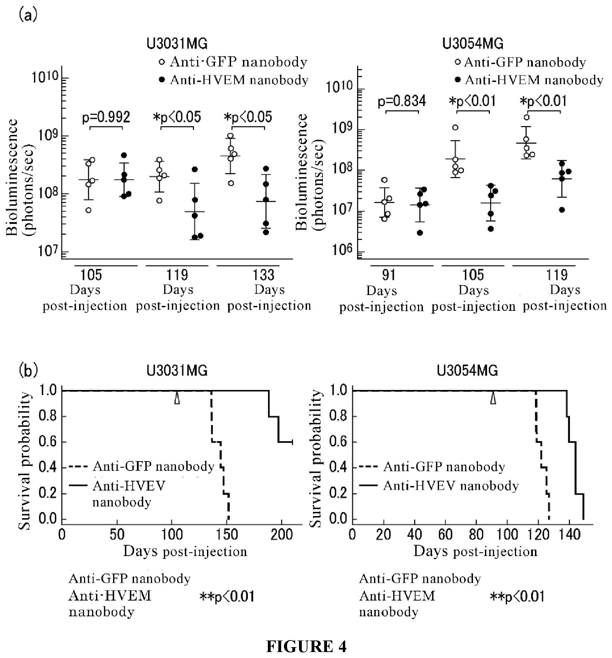

Fig. 4 is a diagram showing the effects of intraperitoneal administration of an alpaca anti-human HVEM antibody on the in vivo proliferation capacity of Mesenchymal subtype GBM cells (number of individuals in each experimental group: n = 5 mice/group, Example 7). Fig. 4(a) is a diagram in which the alpaca anti-human HVEM antibody (VHH-3) was intraperitoneally administered to mice once daily at 10 mg/kg, and in vivo proliferation of Mesenchymal subtype GBM cells (U3031MG or U3054MG) in the mice was quantified at 14 and 28 days after the start of antibody administration. The time initiating antibody administration was day 105 after cell inoculation for tumor-bearing mice with U3031MG cells, and day 91 after cell inoculation for tumor-bearing mice with U3054MG cells. Fig. 4(b) is a diagram showing the survival curve of the mice. The time point at which weight loss of more than 20% and/or the appearance of neurological symptoms were observed in the mouse was defined as the endpoint.

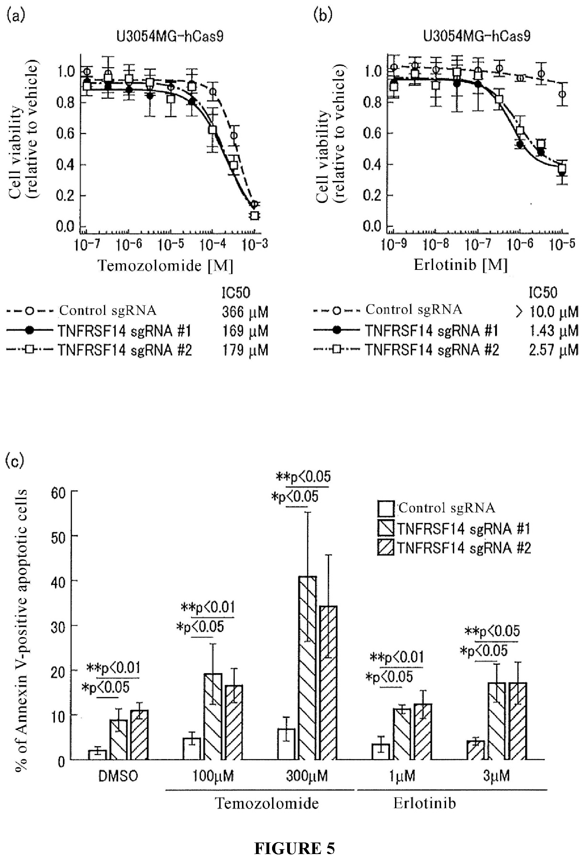

Fig. 5 is a diagram showing that deficiency of the HVEM gene by genome editing increases the sensitivity of Mesenchymal subtype GBM cells to an anticancer agent (temozolomide) and an EGFR inhibitor (erlotinib) (Example 8). Fig. 5(a) is a diagram showing a dose-response curve (biological replicates, n = 3). Fig. 5(b) is a diagram quantifying the frequency of apoptosis (biological replicates, n = 3). Fig. 5(c) shows that apoptotic cells were stained with Annexin V-FITC and 7-AAD, respectively, and apoptosis of Mesenchymal subtype GBM cells was detected by flow cytometry. Cell viability was shown as a ratio relative to the cell viability in the absence of the drug.

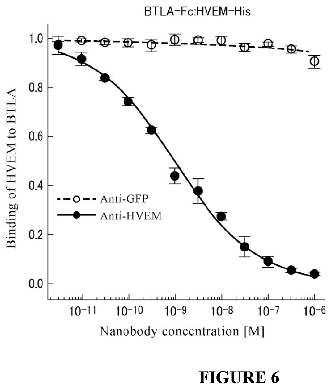

Fig. 6 is a diagram showing that an alpaca anti-human HVEM antibody (VHH-3) inhibits interaction between human BTLA and human HVEM (independent experiments, n = 3, Example 9). A recombinant protein of human HVEM was immobilized on an immunoassay plate, and the alpaca anti-human HVEM antibody (VHH-3) or an anti-GFP antibody (negative control) was reacted over a concentration range of 3 pM to 1 $\mu$M. After reacting with the recombinant protein of human BTLA-Fc, the proportion of the recombinant protein of human BTLA-Fc that reacted with the human HVEM recombinant protein was quantified using an anti-Fc antibody. Binding of HVEM to BTLA was shown as a ratio relative to the binding amount in the absence of the antibody.

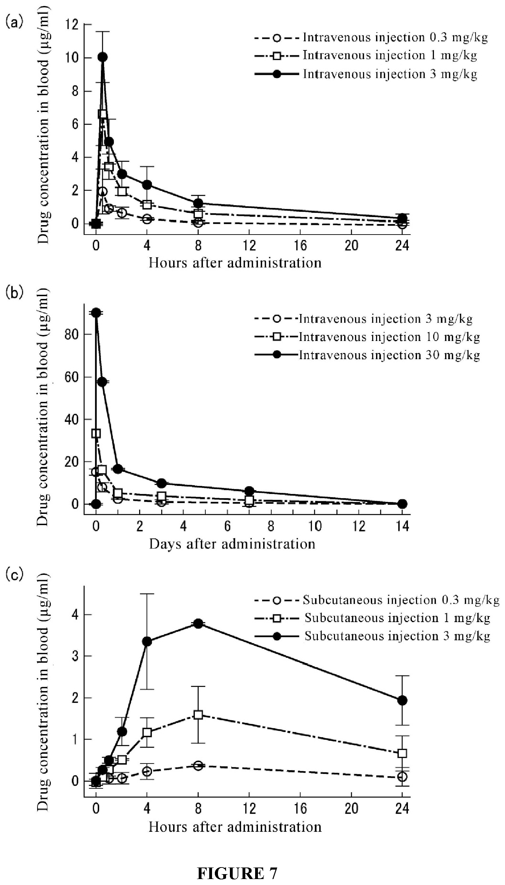

Fig. 7 is a diagram showing the pharmacokinetics of the anti-human HVEM antibody in cynomolgus monkeys (number of individuals in each experimental group: n = 3 animals/group, Example 10). Fig. 7(a) is a diagram showing the antibody concentration in the blood in a case where a humanized anti-human HVEM antibody (HV15) was intravenously injected into cynomolgus monkeys. Fig. 7(b) is a diagram showing the antibody concentration in the blood in a case where a humanized anti-human HVEM antibody (HV80) was intravenously injected into cynomolgus monkeys. Fig. 7(c) is a diagram showing the antibody concentration in the blood in a case where the humanized anti-human HVEM antibody (HV15) was subcutaneously injected into cynomolgus monkeys.

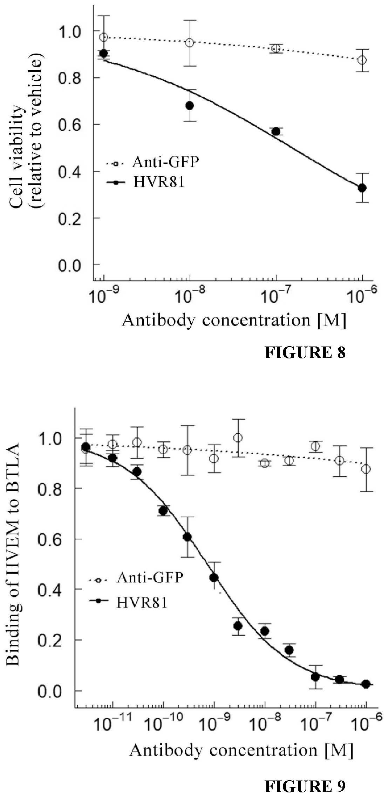

Fig. 8 is a diagram showing that a humanized anti-human HVEM antibody (HVR81) suppresses the proliferation of Mesenchymal subtype GBM cells (U3054MG) in a concentration-dependent manner (Example 12). Each value in the figure indicates an average value and a standard deviation at each antibody concentration. Cell viability was shown as a ratio relative to the cell viability in the absence of the antibodies.

Fig. 9 is a diagram showing that the humanized anti-human HVEM antibody (HVR81) inhibits the interaction between human BTLA and human HVEM in a concentration-dependent manner (Example 13). Each value in the figure indicates an average value and a standard deviation at each antibody concentration. Binding of HVEM to BTLA was shown as a ratio relative to the binding amount in the absence of the antibody.

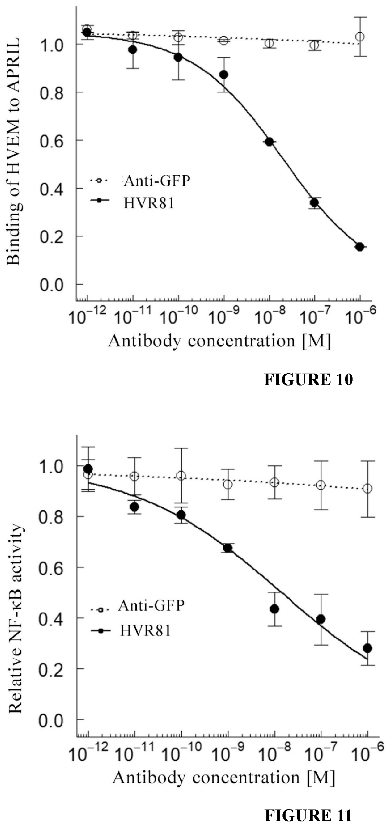

Fig. 10 is a diagram showing that the humanized anti-human HVEM antibody (HVR81) inhibits the interaction between human APRIL and human HVEM in a concentration-dependent manner (Example 13). Each value in the figure indicates an average value and a standard deviation at each antibody concentration. Binding of HVEM to APRIL was shown as a ratio relative to the binding amount in the absence of the antibody.

Fig. 11 is a diagram showing that the humanized anti-human HVEM antibody (HVR81) suppresses NF-kB signaling in Mesenchymal subtype GBM cells (U3054MG) in a concentration-dependent manner (Example 14). Each value in the figure indicates an average value and a standard deviation at each antibody concentration.

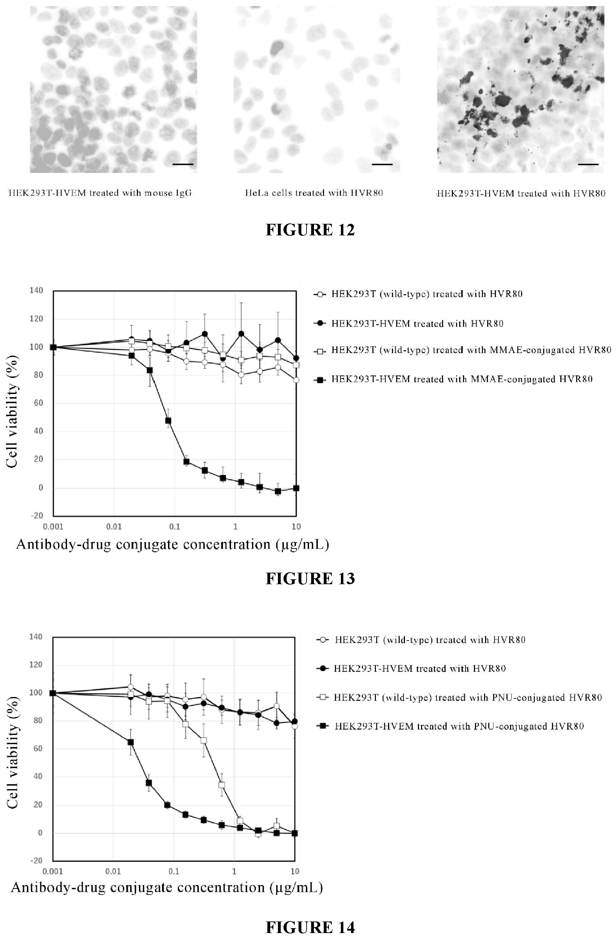

Fig. 12 is a photograph showing the internalization of the humanized anti-human HVEM antibody (HVR80) into HVEM-expressing cells (one side: 160 $\mu$m) (Example 15). Fluorescence emitted by AcidiFluor ORANGE is shown in black, and fluorescence of DAPI, which stains the cell nucleus, is shown in light gray (scale bar: 20 $\mu$m).

Fig. 13 is a diagram showing the suppression of viability of HEK293T-HVEM cells by administration of a humanized anti-human HVEM antibody (HVR80) conjugated with MMAE (Example 16). Each value in the figure indicates the average value and standard deviation of cell viability at each antibody-drug conjugate concentration. Cell viability was shown as a ratio (%) relative to the cell viability in the absence of the antibody-drug conjugate.

Fig. 14 is a diagram showing the suppression of viability of HEK293T-HVEM cells by administration of a humanized anti-human HVEM antibody (HVR80) conjugated with PNU (Example 16). Each value in the figure indicates the average value and standard deviation of cell viability at each antibody-drug conjugate concentration. Cell viability was shown as a ratio (%) relative to the cell viability in the absence of the antibody-drug conjugate.

DETAILED DESCRIPTION OF THE INVENTION

The protein of the present invention is characterized by binding to HVEM. Here, "HVEM" is an abbreviation for Herpes virus entry mediator, and refers to a type I transmembrane protein belonging to the TNF/NGF receptor superfamily. HVEM is also called TNFRSF14 (tumor necrosis factor (TNF) receptor superfamily member 14), CD270, LIGHTR, or ATAR. That is, "HVEM" in the present invention has the same meaning as "HVEM/TNFRSF14".

HVEM is expressed on a variety of tissues and cells, including T cells, B cells, natural killer cells, dendritic cells, hematopoietic cells, and non-hematopoietic cells (parenchymal cells) (Pasero C et al., Curr Opin Pharmacol., 12, 478-485 (2012), Sedy JR and Ramezani-Rad P, Adv. Cancer Res., 42, 145-186 (2019)). Multiple ligands, including TNF related cytokines such as LIGHT and LT$\alpha$ and non-TNF related cytokines such as BTLA, CD160 and SALM5, are known to bind to HVEM.

In the present invention, the human HVEM gene is based on the nucleotide sequence published in HUGO Gene Nomenclature Committee HGNC ID: HGNC: 11912, and the mouse HVEM gene is based on the nucleotide sequence published in Mouse Genome Informatics (MGI): MGI: 2675303. In the present invention, the human HVEM protein is based on the amino acid sequence published in GenBank Accession No. NP_003811.2, and the mouse HVEM protein is based on the amino acid sequence published in GenBank Accession No. NP_849262.1. In the present invention, the mRNA of human HVEM is based on GenBank Accession No. NM_003820.4, and the mRNA of mouse HVEM is based on GenBank Accession No. NM_178931.2.

The protein of the present invention is a protein including at least one immunoglobulin single variable region, that is a protein which is capable of inhibiting the function of HVEM by binding thereto.

As used herein, the term "immunoglobulin variable region" means an antigen-binding site of an antibody, and is composed of a "complementarity determining region" (CDR) and a "framework region" (FR). The complementarity determining regions are also called hypervariable regions (HVR) and are the regions within the variable region that exhibit the highest frequency of variation, whereas the framework regions are regions that are relatively highly conserved.

As the definition of CDR in the present invention, for example, the definition of CDR used in the art, such as Kabat, Chothia, AbM, or contact, can be used. The definition of Kabat is based on sequence changes and is most commonly used (for example, Kabat et al., Sequences of Proteins of Immunological Interest, 5th Ed. Public Health Service, National Institutes of Health, Bethesda, MD. (1991)). Chothia is defined in consideration of the position of the structural loop (for example, Chothia and Lesk, J. Mol. Biol. 196, 901-917 (1987)). AbM is an intermediate definition of Kabat and Chothia structural loops and is used by Oxford Molecular's AbM antibody modeling software.

As used herein, the term "immunoglobulin single variable region" means a single immunoglobulin variable region capable of specifically binding to an epitope of an antigen, rather than paired immunoglobulin variable regions.

A single variable region constituting the protein of the present invention can be characterized by complementarity determining regions 1 to 3 (CDR1, CDR2, and CDR3), and the amino acid sequences of CDR1, CDR2, and CDR3 can be specified by

> an amino acid sequence represented by SEQ ID NO: 1,
> an amino acid sequence represented by SEQ ID NO: 2, and
> an amino acid sequence represented by SEQ ID NO: 3.

The single variable region constituting the protein of the present invention can also be characterized by framework regions 1 to 4 (FR1, FR2, FR3, and FR4), and FR1, FR2, FR3, and FR4 can be specified by a combination of FR1, FR2, FR3, and FR4 selected from the group consisting of the above (i), (ii) and (iii). The single variable region constituting the protein of the present invention can be specified by CDR1 to CDR3 and FR1 to FR4, and in this case, the single variable region constituting the protein of the present invention can be formed by being linked in the order of FR1-CDR1-FR2-CDR2-FR3-CDR3-FR4 from the N-terminus side.

The single variable region constituting the protein of the present invention can also be specified by the single variable region (the polypeptide of the above (v)) of which the sequence was determined in the examples. However, in the present invention, in addition to this, polypeptides that are substantially identical to the polypeptide of the above (v) are also included in the single variable region constituting the protein of the present invention.

A polypeptide substantially identical to the single variable region constituting the protein of the present invention can be represented by a polypeptide selected from the above (vi) and (vii).

In the above (vi), "identity" is used in a meaning including "homology". Here, "identity" is, for example, the degree of identity when sequences to be compared are appropriately aligned, and means the appearance rate (%) of accurate matching of amino acids between the sequences. For identity, for example, the presence of gaps in the sequence and the nature of the amino acids are considered (Wilbur, Natl. Acad. Sci. U.S.A. 80, 726-730 (1983)). The alignment can be performed, for example, by using any algorithm, and specifically, homology search software such as BLAST (Basic local alignment search tool) (Altschul et al., J. Mol. Biol. 215, 403-410 (1990)), FASTA (Peasron et al., Methods in Enzymology 183, 63-69 (1990)), or Smith-Waterman (Meth. Enzym., 164, 765 (1988)) can be used. Furthermore, the identity can be calculated, for example, using the known homology search program as described above, and can be calculated, for example, by using a default parameter in the homology algorithm BLAST (https://blast.ncbi.nlm.nih.gov/Blast.cgi) of the U.S. National Center for Biotechnology Information (NCBI).

The identity in the above (vi) can be at least 80% or more, 85% or more, 90% or more, 91%, 92%, 93%, 94%, 95% or more, 96% or more, 97% or more, 98% or more, or 99% or more with respect to the sequence of the framework region (excluding amino acids corresponding to the humanized region) in the sequence of the above (v).

In the above (vii), "one or more amino acids are deleted, substituted, inserted, and/or added in the sequence of the framework region (excluding amino acids corresponding to the humanized region)" means that, in the sequence of the framework region excluding the amino acid corresponding to the humanized region, for example, a number of amino acids generated by a known method such as a site-directed mutagenesis method or a number of amino acids generated in nature are modified by deletion, substitution, insertion, and/or addition. In the amino acid sequence, the above-mentioned modification may occur continuously or discontinuously, for example.

Examples of the insertion of amino acids in the above (vii) include insertion into the inside of an amino acid sequence. Furthermore, the addition of amino acids in the above (vii) may be, for example, addition to either the N-terminus or the C-terminus of the amino acid sequence, or addition to both the N-terminus and the C-terminus.

The substitution of amino acids in the above (vii) means that an amino acid residue constituting the amino acid sequence is replaced with another type of amino acid residue. The substitution of amino acids in the above (vii) may be, for example, a conservative substitution. "Conservative substitution" means the substitution of one or more amino acids with other amino acids and/or amino acid derivatives not to substantially modify the function of the protein. In the conservative substitution, the amino acid to be substituted and the amino acid after substitution are preferably similar in properties and/or functions, for example. Specifically, it is preferable that indices of hydrophobicity and hydrophilicity, chemical properties such as polarity and charge, or physical properties such as secondary structure are similar. As such, amino acids or amino acid derivatives having similar properties and/or functions are known in the art. Examples of non-polar amino acids (hydrophobic amino acids) include, for example, alanine, valine, isoleucine, leucine, proline, tryptophan, phenylalanine, and methionine. Examples of the polar amino acids (neutral amino acids) include glycine, serine, threonine, tyrosine, glutamine, asparagine, and cysteine. Examples of amino acids having a positive charge (basic amino acids) include arginine, histidine, and lysine, and examples of amino acids having a negative charge (acidic amino acids) include aspartic acid and glutamic acid.

Preferable amino acid modifications in the above (vii) include single amino acid substitution, double amino acid substitution, triple amino acid substitution, quadruple amino acid substitution, quintuple amino acid substitution, sextuple amino acid substitution, or septuple amino acid substitution, and more preferably, the substitution is a conservative substitution.

When the single variable region constituting the protein of the present invention includes the amino acid sequence of SEQ ID NO: 18, the single variable region constituting the protein of the present invention includes the CDR1 having the amino acid sequence represented by SEQ ID NO: 1, the CDR2 having the amino acid sequence represented by SEQ ID NO: 2, the CDR3 having the amino acid sequence represented by SEQ ID NO: 3, the FR1 having the amino acid sequence represented by SEQ ID NO: 5, the FR2 having the amino acid sequence represented by SEQ ID NO: 6, the FR3 having the amino acid sequence represented by SEQ ID NO: 10, and the FR4 having the amino acid sequence represented by SEQ ID NO: 13. In this case, the single variable region can be formed by being linked in the order of FR1-CDR1-FR2-CDR2-FR3-CDR3-FR4 from the N-terminus side. When the single variable region constituting the protein of the present invention includes the amino acid sequence of SEQ ID NO: 18, the sequence of the framework region in the above (vii) (excluding amino acids corresponding to the humanized region) can be:

- a sequence excluding the 1st glutamic acid (Glu) and the 11th leucine (Leu) in the amino acid sequence set forth in SEQ ID NO: 5, that is, a sequence corresponding to the 2nd to 10th and 12th to 25th amino acids in the amino acid sequence set forth in SEQ ID NO: 5;
- a sequence consisting of the 1st to 14th amino acids in the amino acid sequence set forth in SEQ ID NO: 6;
- a sequence excluding the 16th serine (Ser) and the 28th arginine (Arg) in the amino acid sequence set forth in SEQ ID NO: 10, that is, a sequence corresponding to the 1st to 15th, 17th to 27th, and 29th to 39th amino acids in the amino acid sequence set forth in SEQ ID NO: 10; and
- a sequence excluding the 3rd glutamine (Gln) and the 6th leucine (Leu) in the amino acid sequence set forth in SEQ ID No: 13, that is, a sequence corresponding to the 1st, 2nd, 4th, 5th, and 7th to 11th amino acids in the amino acid sequence set forth in SEQ ID NO: 13. When the single variable region constituting the protein of the present invention includes the amino acid sequence of SEQ ID NO: 18, the number of amino acids modified by substitution or the like in the above (vii) is, for example, 1 to 16, 1 to 12, 1 to 11, 1 to 10, 1 to 9, 1 to 8, 1 to 7, 1 to 6, 1 to 5, 1 to 4, 1 to 3, 1 to 2, or 1.

When the single variable region constituting the protein of the present invention includes the amino acid sequence of SEQ ID NO: 19, the single variable region constituting the protein of the present invention includes the CDR1 having the amino acid sequence represented by SEQ ID NO: 1, the CDR2 having the amino acid sequence represented by SEQ ID NO: 2, the CDR3 having the amino acid sequence represented by SEQ ID NO: 3, the FR1 having the amino acid sequence represented by SEQ ID NO: 5, the FR2 having the amino acid sequence represented by SEQ ID NO: 7, the FR3 having the amino acid sequence represented by SEQ ID NO: 10, and the FR4 having the amino acid sequence represented by SEQ ID NO: 13. In this case, the single variable region can be formed by being linked in the order of FR1-CDR1-FR2-CDR2-FR3-CDR3-FR4 from the N-terminus side. When the single variable region constituting the protein of the present invention includes the amino acid sequence of SEQ ID NO: 19, the sequence of the framework region in the above (vii) (excluding the amino acids corresponding to the humanized region) can be:

- a sequence excluding the 1st glutamic acid (Glu) and the 11th leucine (Leu) in the amino acid sequence set forth in SEQ ID NO: 5, that is, a sequence corresponding to the 2nd to 10th and 12th to 25th amino acids in the amino acid sequence set forth in SEQ ID NO: 5;
- a sequence excluding the 5th serine (Ser) in the amino acid sequence set forth in SEQ ID NO: 7, that is, a sequence corresponding to the 1st to 4th and 6th to 14th amino acids in the amino acid sequence set forth in SEQ ID NO: 7;
- a sequence excluding the 16th serine (Ser) and the 28th arginine (Arg) in the amino acid sequence set forth in SEQ ID NO: 10, that is, a sequence corresponding to the 1st to 15th, 17th to 27th, and 29th to 39th amino acids in the amino acid sequence set forth in SEQ ID NO: 10; and

- a sequence excluding the 3rd glutamine (Gln) and the 6th leucine (Leu) in the amino acid sequence set forth in SEQ ID No: 13, that is, a sequence corresponding to the 1st, 2nd, 4th, 5th, and 7th to 11th amino acids in the amino acid sequence set forth in SEQ ID NO: 13. When the single variable region constituting the protein of the present invention includes the amino acid sequence of SEQ ID NO: 19, the number of amino acids modified by substitution or the like in the above (vii) is, for example, 1 to 16, 1 to 12, 1 to 11, 1 to 10, 1 to 9, 1 to 8, 1 to 7, 1 to 6, 1 to 5, 1 to 4, 1 to 3, 1 to 2, or 1.

When the single variable region constituting the protein of the present invention includes the amino acid sequence of SEQ ID NO: 20, the single variable region constituting the protein of the present invention includes the CDR1 having the amino acid sequence represented by SEQ ID NO: 1, the CDR2 having the amino acid sequence represented by SEQ ID NO: 2, the CDR3 having the amino acid sequence represented by SEQ ID NO: 3, the FR1 having the amino acid sequence represented by SEQ ID NO: 5, the FR2 having the amino acid sequence represented by SEQ ID NO: 8, the FR3 having the amino acid sequence represented by SEQ ID NO: 11, and the FR4 having the amino acid sequence represented by SEQ ID NO: 13. In this case, the single variable region can be formed by being linked in the order of FR1-CDR1-FR2-CDR2-FR3-CDR3-FR4 from the N-terminus side. When the single variable region constituting the protein of the present invention includes the amino acid sequence of SEQ ID NO: 20, the sequence of the framework region in the above (vii) (excluding the amino acids corresponding to the humanized region) can be:

- a sequence excluding the 1st glutamic acid (Glu) and the 11th leucine (Leu) in the amino acid sequence set forth in SEQ ID NO: 5, that is, a sequence corresponding to the 2nd to 10th and 12th to 25th amino acids in the amino acid sequence set forth in SEQ ID NO: 5;
- a sequence excluding the 4th glutamine (Gln) and the 5th alanine (Ala) in the amino acid sequence set forth in SEQ ID NO: 8, that is, a sequence corresponding to the 1st to 3rd and 6th to 14th amino acids in the amino acid sequence set forth in SEQ ID NO: 8;
- a sequence excluding the 16th serine (Ser), 19th threonine (Thr), 20th leucine (Leu), 28th arginine (Arg), and 38th alanine (Ala) in the amino acid sequence set forth in SEQ ID NO: 11, that is, a sequence corresponding to the 1st to 15th, 17th, 18th, 21st to 27th, 29th to 37th, and 39th amino acids in the amino acid sequence set forth in SEQ ID NO: 11; and
- a sequence excluding the 3rd glutamine (Gln) and the 6th leucine (Leu) in the amino acid sequence set forth in SEQ ID NO: 13, that is, a sequence corresponding to the 1st, 2nd, 4th, 5th, and 7th to 11th amino acids in the amino acid sequence set forth in SEQ ID NO: 13. When the single variable region constituting the protein of the present invention includes the amino acid sequence of SEQ ID NO: 20, the number of amino acids modified by substitution or the like in the above (vii) is, for example, 1 to 15, 1 to 11, 1 to 10, 1 to 9, 1 to 8, 1 to 7, 1 to 6, 1 to 5, 1 to 4, 1 to 3, 1 to 2, or 1.

Examples of the single variable region constituting the protein of the present invention include a humanized single variable region, preferably a humanized VHH (humanized VHH). VHH is also called a nanobody, and in the present specification, VHH may be referred to as a nanobody.

The protein of the present invention may be a protein having two or more single variable regions in tandem, and the single variable region and the single variable region can be linked by, for example, a peptide linker.

The protein of the present invention may further comprise an Fc region derived from human immunoglobulin in addition to a single variable region, examples of such an Fc region include those derived from human antibodies such as IgG, IgM, IgA, IgE, and IgD, and the Fc region can be preferably an Fc region derived from human IgG4.

When the protein of the present invention includes an Fc region derived from human immunoglobulin, the amino acid sequence of the Fc region may be a polypeptide consisting of the amino acid sequence of the Fc region derived from human immunoglobulin, or may be a polypeptide substantially identical to the polypeptide consisting of the amino acid sequence of the Fc region derived from human immunoglobulin. Examples of the polypeptide substantially identical to the polypeptide consisting of the amino acid sequence of the Fc region derived from human immunoglobulin include, but are not particularly limited to, polypeptides subjected to known modifications that improve the effects expected for the protein of the present invention, and include, for example, polypeptides in which an amino acid is added to the hinge region or polypeptides in which an amino acid in the hinge region is deleted or substituted in order to improve binding affinity, and polypeptides in which an amino acid is substituted in order to prevent IgG4 Fab arm exchange. When the protein of the present invention includes an Fc region derived from human IgG4, non-limiting examples of such an Fc region include a polypeptide represented by an amino acid sequence represented by SEQ ID NO: 14, 15, or 16.

In addition to the single variable region, the protein of the present invention may further comprise a protein other than the Fc region derived from human immunoglobulin, and examples of such peptides include human serum albumin-binding proteins.

When the protein of the present invention includes a single variable region and a human immunoglobulin Fc region, the protein of the present invention may have a structure including two single chains including a single variable region and a human immunoglobulin Fc region, and the two chains may be bound by a disulfide bond. When the protein of the present invention comprises two single chains including a single variable region and a human immunoglobulin Fc region, the two chains may be the same or different, and preferably the two chains are the same chain.

The protein of the present invention can also be provided in the form of an antibody-drug conjugate (ADC). That is, the antibody-drug conjugate of the present invention comprises at least the protein of the present invention and a drug, and in this case, the protein of the present invention may be composed of a single variable region, or may further comprise a protein other than an Fc region derived from human immunoglobulin or an Fc region derived from human immunoglobulin in addition to the single variable region. The drug may be linked to a single variable region or to a region other than the single variable region, and the linkage mode may be direct or via a linker (for example, a peptide or the like cleavable by a predetermined tissue). Examples of the drug include chemotherapeutic agents, and non-limiting examples include anticancer agents (for example, a small molecule anticancer agent).

The protein of the present invention specifically binds to HVEM, and preferably has a low dissociation constant (KD) for human HVEM. The upper limit value (or lower) of KD can be, for example, $10^{-6}$M, $10^{-7}$M, or $10^{-8}$M, and the lower limit value (or higher) is not particularly limited, but can be, for example, $10^{-9}$M or $10^{-10}$M.

The protein of the present invention can be produced, for example, by expressing a polynucleotide encoding them in a host and recovering an expression product. Therefore, according to still another aspect of the present invention, there is provided a host cell into which a polynucleotide encoding a protein of the present invention and the polynucleotide of the present invention or a vector to which the polynucleotide of the present invention is operably linked are introduced. In the present invention, the "polynucleotide" includes DNA and RNA, and further includes modified products and artificial nucleic acids thereof, but is preferably DNA. DNA also includes cDNA, genomic DNA, and chemically synthesized DNA.

The polynucleotide of the present invention is not particularly limited as long as the polynucleotide is expressible in the host used and composed of codons encoding a protein having an HVEM-binding activity, and codon optimization may be performed to make the polynucleotide expressible in the host used or to increase the expression level. Codon optimization can be performed by known methods commonly used in the art.

The proteins of the present invention can be expressed in various host cells, for example, bacterial cells, molds, animal cells, plant cells, baculovirus/insect cells or yeast cells, using these cells. The protein of the present invention can also be expressed in various host cells, for example, animal cells, baculovirus/insect cells, and then secreted to the outside of cells. An expression vector for expressing the protein of the present invention is known, and a vector suitable for various host cells can be used.

When extracting the protein expressed in the host from the cultured microbial cells or cultured cells, after cultivation, the microbial cells or cultured cells are collected by a known method, suspended in an appropriate buffer solution, and the cells are disrupted by means such as ultrasonication, lysozyme, and/or freeze-thawing. Then, a soluble extract is obtained by centrifugation or filtration, and the target protein can be obtained from the obtained extract by appropriately combining known separation and purification methods. In addition, when a protein that is expressed in a host and then secreted to the outside of a cell is extracted, a culture solution after culture is collected, a soluble extract is obtained by centrifugation or filtration, and a target protein can be obtained from the obtained extract by appropriately combining known separation and purification methods. As a known separation and purification method, a method using solubility such as salting out or solvent precipitation, a method mainly using a difference in molecular weight such as dialysis, ultrafiltration, gel filtration, or SDS-PAGE, a method using a difference in charge such as ion exchange chromatography, a method using specific affinity such as affinity chromatography, a method using a difference in hydrophobicity such as reverse phase high performance liquid chromatography, a method using a difference in isoelectric point such as isoelectric point electrophoresis, or the like can be used.

The protein of the present invention has HVEM-binding activity and can inhibit the function of HVEM. Therefore, the protein of the present invention can be used as an active ingredient of an inhibitor of a signaling pathway involving HVEM. Non-limiting examples of inhibition of HVEM signaling pathways include inhibition of the physiological effects of HVEM ligands on HVEM and inhibition of HVEM downstream signals. Here, the HVEM ligands include BTLA, APRIL, LIGHT, or SALM5.

As shown in Examples below, it was confirmed that the binding between BTLA and HVEM was inhibited by the HVEM-binding protein. Here, "BTLA" is an abbreviation for "B- and T-lymphocyte attenuator" (B and T lymphocyte function inhibitory factor), and is also called CD272 (cluster of differentiation 272). BTLA is an inhibitory factor in immune checkpoints and is known to negatively regulate T-cell immune reactions (Watanabe et al. Nat. Immunol., 4, 670-679 (2003), Chen and Mellman, Immunity, 39, 1-10 (2013)). In addition, it is known that antitumor immunity can be enhanced by targeting the interaction between BTLA and HVEM, and that promising results have been obtained in anticancer therapy targeting BTLA-HVEM (Wojciechowicz et al., Eur. J. Med. Chem., 268, 116231 (2024); Sordo-Bahamonde et al., Mol. Cancer 22, 142 (2023)). Therefore, the protein of the present invention (particularly HVR81) can be used as an agent for inducing anticancer immunity and also as an anticancer agent (for example, an agent for treating or preventing solid cancer or a malignant tumor).

As shown in Examples below, it was confirmed that the binding between APRIL and HVEM was inhibited by the HVEM-binding protein. Here, "APRIL" is an abbreviation for "a proliferation-inducing ligand", and is also called TNFSF13 (tumor necrosis factor (TNF) ligand superfamily member 13). That is, "APRIL" in the present invention has the same meaning as "APRIL/TNFSF13". Signaling from APRIL via HVEM has been confirmed to promote the formation of tumors (particularly brain tumors with a high degree of malignancy such as gliomas) (WO 2020/138503). In addition, promising results are expected to be obtained in anticancer therapies targeting the interaction between APRIL and HVEM. Therefore, the protein of the present invention (particularly HVR81) can be used as an anticancer agent (for example, an agent for treating or preventing solid cancer or a malignant tumor).

The HVEM-binding protein of the present invention can inhibit the binding between BTLA and HVEM and can inhibit the binding between APRIL and HVEM. Therefore, the protein of the present invention (particularly, HVR81) can inhibit both a signaling pathway involving BTLA and a signaling pathway involving APRIL among signaling pathways involving HVEM. Therefore, the protein of the present invention (particularly HVR81) is useful as a medicine having two functions of an effect of inducing anticancer immunity and an antitumor effect.

NF-κB signals are involved in cell proliferation and angiogenesis, and are deeply involved in the development of cancer (Nowacka and Jablonska, Cytokine Growth Factor Rev. 61, 38-44 (2021)). In addition, it is known that the NF-κB signal in brain tumor cells is also involved in the transition from the Proneural type, which has a low degree of malignancy, to the Mesenchymal type, which has a higher degree of malignancy (Yamini, Cells 7, 125 (2018), Bhat et al., Cancer Cell 24, 331-346 (2013)). Furthermore, it has been confirmed that APRIL activates NF-κB signaling via HVEM, resulting in promotion of the tumor formation (particularly brain tumors with a high degree of malignancy such as gliomas) (WO 2020/138503). As shown in Examples below, it was confirmed that NF-κB signaling mediated by HVEM is suppressed by the HVEM-binding protein. Therefore, the protein of the present invention (particularly HVR81) can be used as an anticancer agent, for example, an agent for treating or preventing solid cancer or a malignant tumor. The protein of the present invention (particularly HVR81) can also be used as an agent for inhibiting the HVEM signaling pathway, and preferably can be used as an agent for inhibiting the NF-κB signaling pathway mediated by HVEM.

The protein of the present invention has HVEM-binding activity and is capable of suppressing tumor proliferation. In particular, by using the protein of the present invention, it is possible to effectively suppress the formation of malignant tumors (for example, brain tumors with a high degree of malignancy, such as gliomas). Since a substance that inhibits the expression of HVEM or the function of HVEM can be used for the treatment or prevention of solid cancer or a malignant tumor (for example, gliomas), the protein of the present invention can be used as a neutralizing antibody against HVEM, and thus can be used as an active ingredient of a pharmaceutical composition (for example, a composition for the treatment or prevention of solid cancer or a malignant tumor, for example, a composition for treatment or prevention of a brain tumor with a high degree of malignancy, such as gliomas).

According to a preferable aspect of the present invention, the pharmaceutical composition of the present invention can be used for the treatment or prevention of gliomas. Here, "glioma" is a generic term for tumors generated from neuroectodermal tissue of the brain parenchyma, which account for 30 to 40% of primary intracranial tumors and are the most frequent brain tumor. Examples of those included in gliomas are oligodendroglioma, oligoastrocytoma, astrocytoma, and glioblastoma multiforme. "Glioblastoma multiforme" is also called glioblastoma or anaplastic glioma, and is a glioma composed mainly of undifferentiated cells derived from astrocytes. Marked nuclear pleomorphism, necrosis, and vascular endothelial proliferation are observed. Glioblastoma multiforme grows rapidly, invades extensively, and often occurs in the cerebrum of an adult. Glioblastoma multiforme was reported to be classified into four subtypes: Proneural, Neural, Classical, and Mesenchymal (Verhaak R G et al., Cancer Cell, 17, 98-110 (2010)). Since HVEM is highly expressed in glioblastoma multiforme of the Mesenchymal subtype, the composition of the present invention can be preferably used for the treatment and prevention of glioblastoma multiforme (particularly glioblastoma multiforme of Mesenchymal subtype) as well as for reducing the risk of onset of gliomas as described later (particularly glioblastoma multiforme of Mesenchymal subtype).

As shown in Examples described later, by inhibiting the expression or the function of HVEM in glioblastoma multiforme cells, proliferation and cell invasion of glioblastoma multiforme cells could be suppressed. In addition, the protein of the present invention having a human immunoglobulin Fc region was found to have a prolonged plasma half-life. Therefore, the protein of the present invention or the antibody-drug conjugate of the present invention can be administered to a subject (for example, brain tumor patients) whose HVEM expression level exceeds the HVEM expression level of a healthy subject or the HVEM expression level of a normal tissue sample, particularly a subject highly expressing HVEM in the brain among brain tumor patients. The treatment and prevention target of the present invention can also be an HVEM expression-dependent glioma or an HVEM ligand-dependent glioma. Whether the subject highly expresses HVEM, whether the glioma is HVEM dependent, and whether the glioma is HVEM ligand dependent can be evaluated, for example, using brain tissue resected by neurosurgery performed on a brain tumor patient, and can be determined according to known procedures.

The protein of the present invention can be used as a neutralizing antibody against HVEM. The neutralizing antibody against HVEM means an antibody that inhibits the function of HVEM. The neutralizing activity against HVEM can be determined by activities suppressing or inhibiting the function of HVEM, such as suppression of the physiological effect (for example, binding) of HVEM ligand (for example, BTLA, CD160, APRIL, LIGHT or SALM5) on HVEM, or suppression of the HVEM downstream signal (for example, suppression of proliferation or cell invasion of glioblastoma multiforme cells, suppression of NF-$\kappa$B signal), as an index.

The protein of the present invention or the antibody-drug conjugate of the present invention can also be administered to a subject having risk of onset of solid cancer or a malignant tumor, such as gliomas (particularly glioblastoma multiforme), thereby reducing the risk of onset of solid cancer or a malignant tumor. Here, the "subject having a risk of onset of solid cancer or a malignant tumor" means a subject who has no subjective symptom of solid cancer or a malignant tumor or has not been diagnosed with solid cancer or a malignant tumor but is at risk of onset of solid cancer or a malignant tumor in the future, and examples thereof include a person who has not been diagnosed with solid cancer or a malignant tumor, a cancer patient who has undergone resection of solid cancer tissue or malignant tumor tissue, a brain tumor patient who has not been diagnosed with glioma, or a brain tumor patient who has undergone resection of brain tumor tissue. In addition, "reduction in the risk of onset of solid cancer or a malignant tumor" means that the probability of onset of solid cancer or a malignant tumor is reduced, and the reduction in the probability of onset of solid cancer or a malignant tumor improves the prognosis of solid cancer or a malignant tumor (for example, malignant brain tumors).

That is, according to another aspect of the present invention, there is provided a composition for reducing the risk of onset of solid cancer or a malignant tumor (for example, gliomas), comprising as an active ingredient the protein of the present invention or the antibody-drug conjugate of the present invention, and there is also provided a composition for use as an agent for improving prognosis in the treatment of a malignant tumor (for example, a malignant brain tumor), comprising as an active ingredient the protein of the present invention or the antibody-drug conjugate of the present invention.

The composition of the present invention can be provided as a pharmaceutical product. The compositions and pharmaceutical products of the present invention comprise the protein of the present invention or the antibody-drug conjugate of the present invention and a pharmaceutically acceptable carrier.

The compositions and pharmaceutical products of the present invention may comprise an active ingredient other than the protein of the present invention and the antibody-drug conjugate of the present invention, or may be used in combination with an active ingredient other than the protein of the present invention and the antibody-drug conjugate of the present invention or a pharmaceutical product or pharmaceutical composition comprising the same. Examples of active ingredients other than the protein of the present invention and the antibody-drug conjugate of the present invention include anticancer agents including immune checkpoint inhibitors (particularly, anticancer agents intended for the treatment of malignant brain tumors), and non-limiting examples thereof include temozolomide, erlotinib, and nivolumab.

When the protein of the present invention or the antibody-drug conjugate of the present invention is administered to a subject, the administration route is not particularly limited as long as a therapeutic or preventive effect on solid cancer or a malignant tumor, such as gliomas (in particular, glioblastoma multiforme), is obtained, but parenteral administration such as intravenous administration, topical administration (including topical administration or continuous topical administration using a catheter), subcutaneous administration, and intraperitoneal administration is preferable.

As the parenteral administration agent, an appropriate dosage form can be selected depending on an actual administration form, and examples thereof include injections and suppositories. These formulations can be formulated using a pharmaceutically acceptable carrier by procedures commonly practiced in the art (for example, known methods described in the General Rules for Preparations of the Japanese Pharmacopoeia 18th edition, etc.). Examples of the pharmaceutically acceptable carrier include excipients, binders, diluents, additives, flavoring agents, buffering agents, thickeners, coloring agents, stabilizers, emulsifiers, dispersing agents, suspending agents, and preservatives.

The dosage of the HVEM-binding protein or antibody-drug conjugate in the present invention can be determined depending on the type of active ingredient, as well as the sex, age and body weight, and the condition of the administration subject, the dosage form, and the administration route. In the present invention, when the HVEM-binding protein is administered for the purpose of treatment or prevention of solid cancer or a malignant tumor, for example, gliomas (particularly glioblastoma multiforme), the dosage per administration for an adult can be determined, for example, in the range of 0.0001 mg to 1000 mg/kg body weight, but is not limited thereto. The daily dosage of the HVEM-binding protein for an adult can be determined depending on the type of active ingredient, as well as the sex, age and body weight, and the condition of the administration subject, the dosage form, and the administration route, but for example, the above-mentioned dosage of the active ingredient can be administered once per day or divided into 2 to 4 doses per day.

The dosing interval of the HVEM-binding protein or antibody-drug conjugate in the present invention can be determined depending on the type of active ingredient, as well as the sex, age and body weight, and the condition of the administration subject, the dosage form, and the administration route, but for example, administration can be once or twice a day, once or twice a week, or once every 2 to 4 weeks.

The composition of the present invention can be administered not only to humans in need thereof but also to non-human mammals (for example, mice, rats, rabbits, dogs, cats, cattle, horses, pigs, sheep, goats, and monkeys).

In the present invention, "treatment" includes therapeutic benefit, but is not limited thereto, and is used in the sense of including measures taken to obtain a beneficial or desired outcome. In addition, therapeutic benefit means cure or improvement of the disease to be treated. In the present invention, "prevention" is used in the sense of including the reduction of the probability of contracting a disease or the probability of recurrence of a disease. In the present invention, "treatment" and "prevention" are not limited to complete treatment and prevention, and may be treatment and prevention to the extent that those skilled in the art recognize that they have potential therapeutic and preventive effects (for example, delaying the progression of a disease, suppressing the worsening of a disease, or delaying the onset of a disease). In addition, when the disease, which is the treatment and prevention target, is "cancer", the treatment and prevention also include the suppression of metastasis or recurrence of the cancer.

According to another aspect of the present invention, there is provided a method for treating or preventing solid cancer or a malignant tumor, such as gliomas, the method comprising administering an effective amount of the protein of the present invention or a composition containing the same, or an effective amount of the antibody-drug conjugate of the present invention or a composition containing the same, to a subject in need thereof. According to another aspect of the present invention, there is also provided a method for reducing the risk of onset of solid cancer or a malignant tumor, such as gliomas, the method comprising administering an effective amount of the protein of the present invention or a composition containing the same, or an effective amount of the antibody-drug conjugate of the present invention or a composition containing the same, to a subject in need thereof. According to another aspect of the present invention, there is also provided a method for improving prognosis in the treatment of solid cancer or a malignant tumor, for example, a malignant brain tumor, the method comprising administering an effective amount of the protein of the present invention or a composition containing the same, or an effective amount of the antibody-drug conjugate of the present invention or a composition containing the same, to a subject in need thereof. According to another aspect of the present invention, there is also provided a method for inhibition of the HVEM signaling pathway, the method comprising administering an effective amount of the protein of the present invention or a composition comprising the same, or an effective amount of the antibody-drug conjugate of the present invention or a composition comprising the same, to a subject in need thereof. According to another aspect of the present invention, there is also provided a method for inducing anticancer immunity, the method comprising administering an effective amount of the protein of the present invention or a composition comprising the same, or an effective amount of the antibody-drug conjugate of the present invention or a composition comprising the same, to a subject in need thereof. These methods of the present invention can be performed in accordance with the description concerning the protein of the present invention and the composition of the present invention.

According to another aspect of the present invention, there is provided the protein of the present invention or the antibody-drug conjugate of the present invention for use in the treatment or prevention of solid cancer or a malignant tumor, such as gliomas, as therapeutic or preventive medications for solid cancer or a malignant tumor, such as gliomas, or for use in the treatment or prevention method of the present invention. According to another aspect of the present invention, there is also provided the protein of the present invention or the antibody-drug conjugate of the present invention for use in reducing the risk of onset of solid cancer or a malignant tumor, such as gliomas, as a risk-reducing agent for onset of solid cancer or a malignant tumor, such as gliomas, or for use in the risk-reducing method of the present invention. According to another aspect of the present invention, there is also provided the protein of the present invention or the antibody-drug conjugate of the present invention for use in improving prognosis in the treatment of solid cancer or a malignant tumor, for example, a malignant brain tumor, as a prognosis improving agent in the treatment of solid cancer or a malignant tumor, for example, a malignant brain tumor, or for use in the prognosis improving method of the present invention. According to another aspect of the present invention, there is also provided the protein of the present invention or the antibody-drug conjugate of the present invention for use in the inhibition of the HVEM signaling pathway, as an agent for inhibiting the HVEM signaling pathway, or for use in the method for inhibiting the HVEM signaling pathway of the present invention. According to another aspect of the present invention, there is also provided the protein of the present invention or the antibody-drug conjugate of the present invention for use in the induction of anticancer immunity, as an agent for inducing anticancer immunity, or for use in the method for inducing anticancer immunity of the present invention. The above HVEM-binding protein and antibody-drug conjugate can be implemented in accordance with the description concerning the protein of the present invention and the composition of the present invention.

According to another aspect of the present invention, there is provided use of the protein of the present invention or the antibody-drug conjugate of the present invention for the production of an agent for treating or preventing solid cancer or a malignant tumor, such as gliomas, for the production of a risk-reducing agent for onset of solid cancer or a malignant tumor, such as gliomas, for the production of a prognosis improving agent in the treatment of solid cancer or malignant tumor, such as a malignant brain tumor, for the production of an agent for inhibiting the HVEM signaling pathway, or for the production of an agent for inducing anticancer immunity. The above use can be performed in accordance with the description concerning the protein of the present invention and the composition of the present invention.

EXAMPLES

The present invention will be described more specifically based on the following examples, but the present invention is not limited to these examples.

Example 1: Human chimerization of alpaca anti-human HVEM antibody

(1) Binding of alpaca anti-human HVEM antibody (VHH-3) to antigen

Using the VHH-3 antibody, which is an immunoglobulin single variable region, the presence or absence of detection of the VHH-3 antibody remaining on HVEM on the membrane surface of HEK293T cells expressing HVEM was measured. Specifically, HEK293T cells introduced with HVEM and HEK293T cells not expressing HVEM as a negative control were respectively seeded at $1 \times 10^4$ cells/well, fixed with 4% paraformaldehyde, and then reacted with a 6×His-tagged VHH-3 antibody. An anti-histidine tag antibody labeled with horseradish peroxidase HRP (anti-His-tag mAb-HRP-DirecT, MBL Co., Ltd.) was reacted, and the enzymatic reaction product by HRP and its substrate was measured using the ELISA POD Substrate TMB Kit (Nacalai Tesque, Inc.). Each effect size data obtained with respective antibody concentration was fitted to a sigmoid curve using nonlinear regression based on the following formula:

effect size = background + maximum effect size/(1 + 10^(hill coefficient × (logEC50 - log(antibody concentration)))) and the 50% effective concentration (EC50) was determined. As a result, EC50 of VHH-3 was 1.94 nM. VHH-3 has a molecular weight of approximately 12,600 and has been confirmed to exhibit neutralizing activity against human Mesenchymal type glioblastoma.

Table 1 shows the sequences where the three CDR and four FR regions of the alpaca anti-human HVEM antibody were determined according to the definition of AbM.

[Table 1]

| Table 1: Alpaca anti-human HVEM antibody (VHH-3: SEQ ID NO: 17) | | |
|---|---|---|
| Region | SEQ ID NO: | Amino acid sequence |
| CDR1 | 1 | GSIGSIHRWD |
| CDR2 | 2 | TLNSEGGPT |
| CDR3 | 3 | RTVPFEY |
| FR1 | 4 | QVQLVESGGGSVQPGGSLRLSCAAS |
| FR2 | 6 | WYRLCPGKQREWVA |
| FR3 | 9 | YADSVEGRFTISRDNAKNMVYLOMNSLKPEDTAVYRCSA |
| FR4 | 12 | WGRGTQVTVSS |

(2) Design and preparation of alpaca-human chimeric anti-human HVEM antibody (VHH-3_hIgG4Fc)

Alpaca-human chimerization was performed for the alpaca anti-human HVEM antibody (VHH-3) shown in Table 1. The alpaca-human chimeric anti-human HVEM antibody (which may be simply referred to as "VHH-3_hIgG4Fc") shown in Table 2 was designed by fusion with the constant region sequence downstream from the hinge region of human IgG to the C-terminus of VHH-3, and prepared by a known method.

[Table 2]

| Table 2: Alpaca-human chimeric anti-human HVEM antibody (VHH-3_hIgG4Fc: SEQ ID NO: 21) | | | |
|---|---|---|---|
| SEQ ID NO: | Region | SEQ ID NO: | Amino acid sequence |
| 17 | CDR1 | 1 | GSIGSIHRWD |
| | CDR2 | 2 | TLNSEGGPT |
| | CDR3 | 3 | RTVPFEY |
| | FR1 | 4 | QVQLVESGGGSVQPGGSLRLSCAAS |
| | FR2 | 6 | WYRLCPGKQREWVA |
| | FR3 | 9 | YADSVEGRFTISRDNAKNMVYLOMNSLKPEDTAVYRCSA |
| | FR4 | 12 | WGRGTQVTVSS |

(continued)

| SEQ ID NO: | Region | SEQ ID NO: | Amino acid sequence |
|---|---|---|---|
| | | | Table 2: Alpaca-human chimeric anti-human HVEM antibody (VHH-3_hIgG4Fc: SEQ ID NO: 21) |
| | Fc | 14 | GPPCPSCPAPEFLGGPSVFLFPPKPKDTLMISRTPEVT CVVVDVSQEDPEVQFNWYVDGVEVHNAKTKPREEQF NSTYRVVSVLTVLHQDWLNGKEYKCKVSNKGLPSSIEK TISKAKGQPREPQVYTLPPSQEEMTKNQVSLTCLVKG FYPSDIAVEWESNGQPENNYKTTPPVLDSDGSFFLYSR LTVDKSRWQEGNVFSCSVMHEALHNHYTQKSLSLSLGK |

(3) Design and preparation of alpaca-human chimeric anti-human HVEM antibody (VHH-3_hIgG4Fc_S108P)

Referring the alpaca-human chimeric anti-human HVEM antibody (VHH-3_hIgG4Fc) shown in Table 2, an alpaca-human chimeric anti-human HVEM antibody shown in Table 3 (which may be simply referred to as "VHH-3_hIgG4Fc_S108P") was designed by adding four amino acid residues to the hinge region and substituting 108Ser with Pro to prevent IgG4 Fab-arm exchange (the number before the three-letter amino acid abbreviation indicates the position of the amino acid residue from the start of the constant region of a typical hIgG4), and was prepared by a known method.

[Table 3]

| SEQ ID NO: | Region | SEQ ID NO: | Amino acid sequence |
|---|---|---|---|
| | | | Table 3: Alpaca-human chimeric anti-human HVEM antibody (VHH-3_hIgG4Fc_S108P: SEQ ID NO: 22) |
| 17 | CDR1 | 1 | GSIGSIHRWD |
| | CDR2 | 2 | TLNSEGGPT |
| | CDR3 | 3 | RTVPFEY |
| | FR1 | 4 | QVQLVESGGGSVQPGGSLRLSCAAS |
| | FR2 | 6 | WYRLCPGKQREWVA |
| | FR3 | 9 | YADSVEGRFTISRDNAKNMVYLQMNSLKPEDTAVYRCSA |
| | FR4 | 12 | WGRGTQVTVSS |
| | Fc | 15 | ESKYGPPCPPCPAPEFLGGPSVFLFPPKPKDTLMISRTPEVT CVVVDVSQEDPEVQFNWYVDGVEVHNAKTKPREEQF NSTYRVVSVLTVLHQDWLNGKEYKCKVSNKGLPSSIEK TISKAKGQPREPQVYTLPPSQEEMTKNQVSLTCLVKG FYPSDIAVEWESNGQPENNYKTTPPVLDSDGSFFLYSR LTVDKSRWQEGNVFSCSVMHEALHNHYTQKSLSLSLGK |

Example 2: Humanization of alpaca-human chimeric anti-human HVEM antibody

(1) Design and preparation of humanized antibody (HV80) of alpaca-human chimeric anti-human HVEM antibody

Humanization was performed on the alpaca-human chimeric anti-human HVEM antibody (VHH-3_hIgG4Fc) obtained in Example 1. The humanized anti-human HVEM antibody (which may be simply referred to as "humanized VHH-3_hIgG4Fc" or "HV80") shown in Table 4 was designed by humanizing 6 amino acids of VHH-3_hIgG4Fe.

Incidentally, humanization was performed by requesting GenScript Biotech Corporation, and a humanized antibody clone considered to be most effective was selected from the viewpoint of binding affinity toward HVEM and expression efficiency in mammalian cells. The humanized anti-human HVEM antibody (HV80) has a molecular weight of about 76,000.

[Table 4]

| SEQ ID NO: | Region | SEQ ID NO: | Amino acid sequence |
|---|---|---|---|
| Table 4: Humanized anti-human HVEM antibody (HV80: SEQ ID NO: 23) | | | |
| 18 | CDR1 | 1 | GSIGSIHRWD |
| | CDR2 | 2 | TLNSEGGPT |
| | CDR3 | 3 | RTVPFEY |
| | FR1 | 5 | EVQLVESGGGLVQPGGSLRLSCAAS |
| | FR2 | 6 | WYRLCPGKQREWVA |
| | FR3 | 10 | YADSVEGRFTISRDNSKNMVYLQMNSLRPEDTAVYRCSA |
| | FR4 | 13 | WGQGTLVTVSS |
| | Fc | 14 | GPPCPSCPAPEFLGGPSVFLFPPKPKDTLMISRTPEVTCVV VDVSQEDPEVQFNWYVDGVEVHNAKTKPREEQFNSTYRVV SVLTVLHQDWLNGKEYKCKVSNKGLPSSIEKTISKAKGQPRE PQVYTLPPSQEEMTKNQVSLTCLVKGFYPSDIAVEWESNGQ PENNYKTTPPVLDSDGSFFLYSRLTVDKSRWQEGNVFSCSV MHEALHNHYTQKSLSLSLGK |

(2) Design and preparation of humanized antibody (HV15) of alpaca anti-human HVEM antibody

Referring the humanized anti-human HVEM antibody (HV80) obtained in the above (1), a humanized anti-human HVEM antibody (which may be simply referred to as "HV15") shown in Table 5 was designed by substituting the amino acid sequence of the Fc region (SEQ ID NO: 14) with an amino acid sequence consisting of six histidines. The humanized anti-human HVEM antibody (HV15) has a molecular weight of about 13,400.

[Table 5]

| SEQ ID NO: | Region | SEQ ID NO: | Amino acid sequence |
|---|---|---|---|
| Table 5: Humanized anti-human HVEM antibody (HV15: SEQ ID NO: 28) | | | |
| 18 | CDR1 | 1 | GSIGSIHRWD |
| | CDR2 | 2 | TLNSEGGPT |
| | CDR3 | 3 | RTVPFEY |
| | FR1 | 5 | EVQLVESGGGLVQPGGSLRLSCAAS |
| | FR2 | 6 | WYRLCPGKQREWVA |
| | FR3 | 10 | YADSVEGRFTISRDNSKNMVYLQMNSLRPEDTAVYRCSA |
| | FR4 | 13 | WGQGTLVTVSS |
| | | 27 | HHHHHH |

(3) Design and preparation of humanized antibody (HVs80) of alpaca-human chimeric anti-human HVEM antibody

Referring the humanized anti-human HVEM antibody (HV80) obtained in the above (1), a humanized anti-human HVEM antibody (which may be simply referred to as "humanized VHH-3_hIgG4Fc Cys->Ser" or "HVs80") shown in Table 6 was designed by substituting 40Cys in the amino acid sequence of FR2 (SEQ ID NO: 6) with Ser (the number before the three-letter amino acid abbreviation indicates the position of the amino acid residue according to the definition by Kabat et al.), and was prepared by a known method.

[Table 6]

Table 6: Humanized anti-human HVEM antibody (HVs80: SEQ ID NO: 24)

| SEQ ID NO: | Region | SEQ ID NO: | Amino acid sequence |
|---|---|---|---|
| 19 | CDR1 | 1 | GSIGSIHRWD |
| | CDR2 | 2 | TLNSEGGPT |
| | CDR3 | 3 | RTVPFEY |
| | FR1 | 5 | EVQLVESGGGLVQPGGSLRLSCAAS |
| | FR2 | 7 | WYRLSPGKQREWVA |
| | FR3 | 10 | YADSVEGRFTISRDNSKNMVYLQMNSLRPEDTAVYRCSA |
| | FR4 | 13 | WGQGTLVTVSS |
| | Fc | 14 | GPPCPSCPAPEFLGGPSVFLFPPKPKDTLMISRTPEVTCVV VDVSQEDPEVQFNWYVDGVEVHNAKTKPREEQFNSTYRVV SVLTVLHQDWLNGKEYKCKVSNKGLPSSIEKTISKAKGQPRE PQVYTLPPSQEEMTKNQVSLTCLVKGFYPSDIAVEWESNGQ PENNYKTTPPVLDSDGSFFLYSRLTVDKSRWQEGNVFSCSV MHEALHNHYTQKSLSLSLGK |

(4) Design and preparation of humanized antibody (HVR80) of alpaca anti-human HVEM antibody (VHH-3)

From the V/D/J junction sequences contained in the human germline sequence database, YAC-5+/D6-25/JH4d was extracted as the germline sequence encoding the amino acid sequence with the highest homology to the amino acid sequence of VHH-3. In the amino acid sequences encoded by YAC-5+, within the region corresponding to the second framework of the human heavy chain antibody, amino acid residues 37V, 44G, and 45L, which possess hydrophobic side chains (the numbers before the one-letter amino acid abbreviations indicate the positions of the amino acid residues according to the definition by Kabat et al.), were substituted with amino acid residues 37Y, 44Q, and 45R, which possess hydrophilic side chains and are present in the second framework sequence of VHH-3, contributing to the stability of the VHH as a single chain.

The CDR sequences determined according to definitions of AbM and the others within VHH-3 were transplanted between each of the first to fourth framework sequences to construct a prototype of a humanized VHH-3 antibody candidate, and the codons were optimized from the gene encoding the amino acid sequence to facilitate translation into protein in human cells. This humanized VHH-3 antibody candidate gene sequence was synthesized as an artificial gene, and a plasmid was constructed by insertion into a pcDNA3.4 vector with a construct for expressing an antibody in which the constant region sequence of human IgG4 downstream from the hinge region (with 108Ser substituted with Pro to prevent IgG4 Fab-arm exchange, and here, the number before the three-letter amino acid abbreviation indicates the position of the amino acid residue from the start of the constant region of a typical hIgG4) was fused to the C-terminus of VHH-3 downstream of the Igκ signal peptide sequence. In the prototype, 16 amino acids in the FR sequence in the VHH-3 region were substituted with the same amino acid as in the human sequence. The plasmid was transfected into high-density cultured Expi293F™ cells using ExpiFectamine™ 293 reagent. ExpiFectamine™ 293 transfection enhancer was added to improve transfection, cell viability, and protein expression 16-18 hours later. Seven days after transfection, the culture solution was collected. The antibody was adsorbed using Protein G bound to the surface of Sepharose beads, and was then eluted with 0.1 M glycine buffer (pH 2.7), immediately neutralized with one-tenth volume of 1 M Tris-HCl buffer (pH 10). The solution was then substituted with PBS to obtain the prototype of the antibody.

Until notable antigen-binding activity of the humanized VHH-3 candidate was obtained, back-mutations from the humanized sequence prototype were repeatedly performed, and finally, remarkable antigen-binding activity was acquired for the first time with the humanized anti-human HVEM antibody shown in Table 7 (which may be simply referred to as "HVR80"), which has a sequence in which 11 amino acids of the alpaca-human chimeric anti-human HVEM antibody (VHH-3_hIgG4Fc) sequence were substituted. The molecular weight of the HVR80 is about 77,000.

[Table 7]

| SEQ ID NO: | Region | SEQ ID NO: | Amino acid sequence |
|---|---|---|---|
| Table 7: Humanized anti-human HVEM antibody (HVR80: SEQ ID NO: 25) | | | |
| 20 | CDR1 | 1 | GSIGSIHRWD |
| | CDR2 | 2 | TLNSEGGPT |
| | CDR3 | 3 | RTVPFEY |
| | FR1 | 5 | EVQLVESGGGLVQPGGSLRLSCAAS |
| | FR2 | 8 | WYRQAPGKQREWVA |
| | FR3 | 11 | YADSVEGRFTISRDNSKNTLYLQMNSLRPEDTAVYRCAA |
| | FR4 | 13 | WGQGTLVTVSS |
| | Fc | 16 | GPPCPPCPAPEFLGGPSVFLFPPKPKDTLMISRTPEVTCVV VDVSQEDPEVQFNWYVDGVEVHNAKTKPREEQFNSTYRVV SVLTVLHQDWLNGKEYKCKVSNKGLPSSIEKTISKAKGQPRE PQVYTLPPSQEEMTKNQVSLTCLVKGFYPSDIAVEWESNGQ PENNYKTTPPVLDSDGSFFLYSRLTVDKSRWQEGNVFSCSV MHEALHNHYTQKSLSLSLGK |

(5) Design and preparation of humanized antibody (HVR81) of alpaca anti-human HVEM antibody (VHH-3)

Referring the humanized anti-human HVEM antibody (HVR80) shown in Table 7, a humanized anti-human HVEM antibody (which may be simply referred to as "HVR81") shown in Table 8 was designed by adding four amino acid residues to the hinge region, and was prepared by a known method.

[Table 8]

| SEQ ID NO: | Region | SEQ ID NO: | Amino acid sequence |
|---|---|---|---|
| Table 8: Humanized anti-human HVEM antibody (HVR81: SEQ ID NO: 26) | | | |
| 20 | CDR1 | 1 | GSIGSIHRWD |
| | CDR2 | 2 | TLNSEGGPT |
| | CDR3 | 3 | RTVPFEY |
| | FR1 | 5 | EVQLVESGGGLVQPGGSLRLSCAAS |
| | FR2 | 8 | WYRQAPGKQREWVA |
| | FR3 | 11 | YADSVEGRFTISRDNSKNTLYLQMNSLRPEDTAVYRCAA |
| | FR4 | 13 | WGQGTLVTVSS |
| | Fc | 15 | ESKYGPPCPPCPAPEFLGGPSVFLFPPKPKDTLMISRTPEVT CVVVDVSQEDPEVQFNWYVDGVEVHNAKTKPREEQFNSTY RVVSVLTVLHQDWLNGKEYKCKVSNKGLPSSIEKTISKAKGQ PREPQVYTLPPSQEEMTKNQVSLTCLVKGFYPSDIAVEWES NGQPENNYKTTPPVLDSDGSFFLYSRLTVDKSRWQEGNVF SCSVMHEALHNHYTQKSLSLSLGK |

Example 3: Evaluation of the degree of humanization of antibody sequences

The degree of humanization was determined for sequences of the alpaca anti-human HVEM antibody (VHH-3), the alpaca-human chimeric anti-human HVEM antibody (VHH-3_hIgG4Fc), and the humanized anti-human HVEM antibody (HVR80) in accordance with the integrated antibody sequence and structure analysis program (version 3.4.1) provided by Ebisu Co. Actually, the degree of humanization was determined by annotating the amino acid sequences of the single variable region of VHH-3 (SEQ ID NO: 17), the humanized anti-human HVEM antibody (HVs80) (SEQ ID NO: 19), and the humanized anti-human HVEM antibodies (HVR80 and HVR81) (SEQ ID NO: 20) using abYsis (http://www.abysis.org/abysis/index.html, Swindells et al., J Mol Biol., 429, 356-364 (2017)).

The results were as shown in Fig. 1. It was shown that the single variable region of HVs80, as well as the single variable regions of HVR80 and HVR81, have a higher degree of humanization compared to VHH-3, and that the single variable regions of HVR80 and HVR81 have a higher degree of humanization compared to the single variable region of HVs80.

Example 4: Evaluation of binding affinity of antibody to HVEM

(1) Measurement of binding affinity of humanized anti-human HVEM antibody (HVR80) to human HVEM

Binding affinity to human HVEM was measured by surface plasmon resonance using BIAcore T200. A humanized anti-human HVEM antibody candidate solution diluted to 30 µg/ml with a running buffer (10 mM HEPES pH 7.5, 150 mM NaCl, 0.1% Tween 20) was applied at a flow rate of 10 µl/min for 2 minutes on a CM5 sensor chip on which an anti-human IgG antibody was immobilized by an amine coupling method, whereby the humanized anti-human HVEM antibody candidate was captured on the sensor chip. Furthermore, human HVEM extracellular domain recombinant protein (Sino Biological Japan Inc. 10334-H08H) diluted in the running buffer was applied as an analyte at a flow rate of 10 µl/min for 2 minutes, and the Ka value was measured. Thereafter, the Kd value was measured in 2 minutes after switching to the running buffer comprising no antibody. KD values were calculated by Kd/Ka.

(2) Measurement of binding affinity of alpaca anti-human HVEM antibody (VHH-3), alpaca-human chimeric anti-human HVEM antibody (VHH-3_hIgG4Fc), and humanized anti-human HVEM antibody (HV80, HVR81) to human HVEM

Regarding the VHH-3, a human HVEM extracellular domain recombinant protein (Sino Biological Japan Inc. 10334-H08H) diluted in the running buffer was applied as an analyte at a flow rate of 10 µl/min for 2 minutes on an SA sensor chip immobilized with the VHH-3 tagged C-terminally with biotinylated Avitag to measure the Ka value. Thereafter, the Kd value was measured in 2 minutes after switching to the running buffer comprising no antibody. KD values were calculated by Kd/Ka. For VHH-3_hIgG4Fc, HV80, and HVR81, KD values were calculated in the same manner as in the above (1) except that the humanized anti-human HVEM antibody candidate solution was changed to a solution containing VHH-3_hIgG4Fc, HV80, or HVR81.

(3) Results

The results are as shown in Table 9.

[Table 9]

| Table 9: Binding affinity of antibodies to HVEM | |
|---|---|
| Antibody | KD value |
| VHH-3 | 19 nM |
| VHH-3_hIgG4Fc | 67 nM |
| HV80 | 102 nM |
| HVR80 | 59 nM |
| HVR81 | 32 nM |

HVR80 and HVR81 were shown to have increased HVEM binding affinity compared to HV80.

Example 5: Effects of introduction of alpaca anti-human HVEM antibody gene on proliferation of Mesenchymal subtype cells

(1) Gene introduction by lentivirus

The CMV promoter, the alpaca anti-human HVEM antibody (VHH-3) gene (SEQ ID NO: 29) or the anti-GFP antibody (negative control) gene (SEQ ID NO: 30), and the bGH polyA sequence were inserted between the BglII site and the XbaI site of the pENTR4 plasmid, and an LR reaction was performed with the CS-RfA-EG plasmid. In addition to the LR reaction generating plasmid, pCAG-HIVgp and pCMV-RSV-G-Rev were transfected into HEK293FT cells with Lipofectamine 2000 Reagent (Thermo Fisher Scientific) to generate lentiviruses. The culture supernatant comprising lentiviral particles was collected and concentrated using a Lenti-X Concentrator (TAKARA Bio). The concentrated lentivirus was suspended in serum-free media and used to infect GBM cells. Since GBM cells expressing antibody genes are labeled with EGFP, EGFP-positive cells were sorted by cell sorter. The amino acid sequence of the expressed alpaca anti-human HVEM antibody (VHH-3) was SEQ ID NO: 34, and the amino acid sequence of the anti-GFP antibody (negative control) was SEQ ID NO: 35.

(2) Cell proliferation assay

Mesenchymal subtype GBM cells into which each antibody gene was introduced, obtained in the above (1), were seeded at a density of $1 \times 10^3$ cells/well. The number of viable cells was quantified by a CCK-8 assay at each time point 3, 5, or 7 days after seeding, and normalized with the quantitative value at the time of seeding.

(3) Results

The results were as shown in Fig. 2. It was shown that in Mesenchymal subtype GBM cells into which the alpaca anti-human HVEM antibody (VHH-3) gene was introduced, cell proliferation was significantly suppressed as compared with the negative control.

Example 6: Effects of alpaca anti-human HVEM antibody on proliferation and cell invasion of Mesenchymal subtype cells

(1) Cell proliferation assay

Mesenchymal subtype GBM cells were seeded at a density of $1 \times 10^3$ cells/well and treated with alpaca anti-human HVEM antibody (VHH-3) or anti-KLH antibody (negative control) at a concentration (1 μM). The number of viable cells was quantified by a CCK-8 assay at each time point 3, 5, or 7 days after seeding, and normalized with the quantitative value at the time of seeding to obtain a proliferation curve. In addition, the number of viable cells was quantified by a CCK-8 assay at the time point 7 days after seeding and normalized with the quantitative value under the antibody untreated condition to obtain a dose-response curve.

(2) Cell invasion Assay

Mesenchymal subtype GBM cells were treated with CFSE, and viable cells were fluorescently labeled. CFSE-labeled cells were seeded at a density of $1 \times 10^4$ cells/well in a low cell-attachment microplate and cultured for 24 hours to produce spheroids of Mesenchymal subtype GBM cells. Mouse brains sliced at a thickness of 300 to 500 μm were cultured, and spheroids of Mesenchymal subtype GBM cells were placed on mouse brain slices to observe the morphology of the spheroids by fluorescence microscopy. Spheroids of Mesenchymal subtype GBM cells were treated with an alpaca anti-human HVEM antibody (VHH-3) or an anti-GFP antibody (negative control) at a concentration (1 μM) on mouse brain slices and cultured for 2 days, and the morphology of the spheroids was observed with a fluorescence microscope. The invasion area of spheroids was quantified, and the invasion was evaluated by normalizing to the size of the spheroids prior to antibody treatment.

(3) Results

The results were as shown in Fig. 3. It was shown that in Mesenchymal subtype GBM cells treated with the alpaca anti-human HVEM antibody (VHH-3), cell proliferation was suppressed as compared with the negative control. It was also shown that invasion of spheroids of Mesenchymal subtype GBM cells (U3031MG and U3054MG) into mouse brain was significantly suppressed by the alpaca anti-human HVEM antibody (VHH-3).

Example 7: Effects of intraperitoneal administration of alpaca anti-human HVEM antibody on in vivo proliferative capacity of Mesenchymal subtype GBM cells

(1) Animal experiments

Mesenchymal subtype GBM cells (U3031MG or U3054MG) were injected into brains of BALB/c-nu/nu mice with $1 \times 10^5$ cells. The tumor size was quantified by bioluminescence imaging before antibody administration, and the subjects were divided into two groups such that the amount of luminescence was equalized. The timing of antibody administration initiation was started 105 days after cell inoculation for the tumor-bearing mouse derived from U3031MG cells and 91 days after cell inoculation for the tumor-bearing mouse derived from U3054MG cells, and an alpaca anti-human HVEM antibody (VHH-3) or an anti-GFP antibody (negative control) was intraperitoneally administered to the mice at a dose of 10 mg/kg every day. After 14 and 28 days from the start of administration of the antibody, bioluminescence imaging was performed to quantify the tumor size, and the effect of the antibody on the in vivo proliferation capacity was examined. The time point at which a body weight loss of 20% or more and/or neurological symptoms were observed was defined as the endpoint for the mouse, and survival time analysis was performed. It is noted that, under the conditions of this experiment, the engraftment rate of GBM cells was 100%, and therefore no individuals were excluded due to lack of engraftment.

(2) Results

The results were as shown in Fig. 4. In the group administered the alpaca anti-human HVEM antibody (VHH-3), it was shown that tumor size was significantly reduced and survival period was significantly prolonged compared to the negative control.

Example 8: Influence of HVEM gene deficiency on anticancer agents

(1) Genome editing with CRISPR-Cas9 system

The DNA sequence of sgRNA targeting the human TNFRSF14 gene (SEQ ID NO: 31 and SEQ ID NO: 32) or the DNA sequence of negative control sgRNA (SEQ ID NO: 33) was designed downstream of the hU6 promoter in the lentiCRISPR v2 plasmid. In addition to the generated plasmid, pCAG-HIVgp and pCMV-VSV-G-RSV-Rev were transfected into HEK293FT cells with Lipofectamine 2000 Reagent (Thermo Fisher Scientific) to generate lentiviruses. The culture supernatant comprising lentiviral particles was collected and concentrated using a Lenti-X Concentrator (TAKARA Bio). The concentrated lentivirus was suspended in serum-free medium and used to infect GBM cells (U3054MG) to obtain genome-edited GBM cells (U3054MG-hCas9). GBM cells deficient in the human TNFRSF14 gene were obtained by genome editing by fractionating each single cell by a cell sorter, culturing each, and confirming the DNA sequence of the human TNFRSF14 gene.

(2) Cell proliferation assay

GBM cells deficient in the human TNFRSF14 gene obtained in the above (1) were seeded, and treated with DMSO (negative control), an anticancer agent (temozolomide), or an EGFR inhibitor (erlotinib) at each concentration described in the drawing. After the cells were cultured for 7 days, the number of viable cells was quantified by a CCK-8 assay and normalized with the quantitative value under the drug-untreated condition to obtain a dose-response curve.

(3) Apoptosis detection assay

GBM cells deficient in the human TNFRSF14 gene obtained in the above (1) were treated with DMSO (negative control), an anticancer agent (temozolomide), or an EGFR inhibitor (erlotinib) at each concentration shown in the drawing, and cultured for 7 days. Cells under each condition were collected and stained with Annexin V-FITC and 7-AAD. Apoptosis of Mesenchymal subtype GBM cells was detected by flow cytometer.

(4) Results

The results were as shown in Fig. 5. Mesenchymal subtype GBM cells deficient in the HVEM gene were shown to have significantly increased sensitivity to anticancer agents (temozolomide) and EGFR inhibitors (erlotinib).

Example 9: Effects of alpaca anti-human HVEM antibody on interaction between human BTLA and human HVEM

(1) Sandwich ELISA

A recombinant protein of human HVEM was immobilized on an immunoassay plate, and the alpaca anti-human HVEM antibody (VHH-3) or an anti-GFP antibody (negative control) was reacted for 1 hour. After washing, the recombinant protein of human BTLA-Fc was reacted for 1 hour. After washing, the HRP-labeled anti-Fc antibody was reacted for 30 minutes. After further washing, 100 $\mu$L of a TMB substrate solution was added thereto for reaction for 5 minutes. 50 $\mu$L of 2 N sulfuric acid was added to stop the enzymatic reaction. The absorbance at 450 nm was measured with a plate reader, and the amount of the human BTLA-Fc recombinant protein binding to the human HVEM recombinant protein was quantified.

(2) Results

The results were as shown in Fig. 6. It was shown that the alpaca anti-human HVEM antibody (VHH-3) inhibits the binding between human BTLA and human HVEM in a concentration-dependent manner. That is, according to the alpaca anti-human HVEM antibody (VHH-3), it was suggested that, along with the anti-glioma effect through inhibition of HVEM, inhibition of the suppression of immune responses through inhibition of BTLA can also be achieved.

Example 10: Pharmacokinetics of humanized anti-human HVEM antibody in cynomolgus monkeys

(1) Animal experiments

Heparinized blood was collected before administering the antibody to the cynomolgus monkeys. Humanized anti-human HVEM antibody (HV15) at each concentration shown in the drawing (0.3 mg/kg, 1 mg/kg, 3 mg/kg) was administered to cynomolgus monkeys by intravenous injection or subcutaneous injection, and heparinized blood samples were collected from each individual at 30 minutes, 1 hour, 2 hours, 4 hours, 8 hours, and 24 hours after antibody administration. In addition, humanized anti-human HVEM antibody (HV80) at each concentration (3 mg/kg, 10 mg/kg, 30 mg/kg) was administered to cynomolgus monkeys by intravenous injection, and heparinized blood samples were collected from each individual at 1 hour, 6 hours, 24 hours, 3 days, 7 days, and 14 days after antibody administration. After blood collection, plasma components were extracted. It is noted that this study was conducted at Ina Research Inc.

(2) Sandwich ELISA

A recombinant protein of human HVEM was immobilized on an immunoassay plate, and the plasma derived from cynomolgus monkey obtained in the above (1) was reacted for 1 hour. After washing, the HRP-labeled anti-His tag antibody or anti-Fc antibody was reacted for 30 minutes. Further, washing was performed, and 100 $\mu$L of a TMB substrate solution was added thereto for reaction for 5 minutes. 50 $\mu$L of 2 N sulfuric acid was added to stop the enzymatic reaction. The absorbance at 450 nm was measured with a plate reader to quantify the amount of antibody in plasma and to determine the antibody concentration in the blood.

(3) Results

The results were as shown in Fig. 7. It was shown that the humanized anti-human HVEM antibody (HV15) and the humanized anti-human HVEM antibody (HV80) are maintained in vivo, and that the humanized anti-human HVEM antibody (HV80), compared to the humanized anti-human HVEM antibody (HV15), shows an extended plasma half-life. For HV15, subcutaneous injection was shown to have a mild but prolonged plasma half-life compared to intravenous injection.

Example 11: Single dose toxicity study of humanized anti-human HVEM antibody in cynomolgus monkeys

Toxicological changes were examined following a single intravenous administration of humanized anti-human HVEM antibody (HVs80) at 100 mg/kg or 300 mg/kg to groups of two male cynomolgus monkeys each. It is noted that this study was conducted at Ina Research Inc.

(1) Animal welfare

This study was properly conducted in accordance with the Test Plan examined by the Institutional Animal Care and Use Committee (IACUC) of the Test Facility in compliance with the "the Act on Welfare and Management of Animals" and related guidelines. The test facility is certified by AAALAC International.

(2) Test methods

Cynomolgus monkeys (4 males, age at administration: 3 years, body weight at administration: 2.68 to 3.58 kg) were administered the test substance HVs80 by intravenous injection into the tail vein at the dosage shown in Table 10 (administration time: 1 hour, number of administrations: once). The dosages in this study were set at 3.3 times and 10 times the dosage of 30 mg/kg, based on the favorable results obtained at 30 mg/kg in a previously conducted pharmacokinetic study.

[Table 10]

| Table 10: Dosage | | | | | |
|---|---|---|---|---|---|
| Group | Test substance | Dose (mg/kg) | Concentration (mg/mL) | Administration volume (mL/kg) | Administration rate (mL/kg/h) |
| Low-dose group | HVs80 | 100 | 3.33 | 30 | 30 |
| High-dose group | HVs80 | 300 | 10 | 30 | 30 |

(3) Observation

The observation period was defined from the day of animal acquisition (transfer) to the end of the study (day 14 post-administration), with the day of administration designated as Day 1. The observation items included general condition, body weight, and food consumption.

(4) Blood test

Hematological tests (coagulation tests and complete blood count) and blood biochemical tests were performed. Blood was collected from the femoral vein on Days 2, 8, and 14 after administration, as well as before administration (during the acclimation period).

In the complete blood count, the following parameters were measured using a multi-parameter automated hematology analyzer XN-2000 (Sysmex Corporation): red blood cell count (RBC), hemoglobin concentration (HGB), hematocrit (HCT), mean corpuscular volume (MCV), mean corpuscular hemoglobin (MCH), mean corpuscular hemoglobin concentration (MCHC), reticulocyte percentage (RET%) and absolute number (RET#), platelet count (PLT), white blood cell count (WBC), and white blood cell classification[a] percentage (Diff WBC%) and absolute number (Diff WBC #) [a] neutrophils (NEUT), lymphocytes (LYMPH), monocytes (MONO), eosinophils (EO), and basophils (BASO).

In the coagulation test, prothrombin time (PT) and activated partial thromboplastin time (APTT) were measured by a fully automated blood coagulation analyzer, CA-510 (Sysmex Corporation).

In the blood biochemical tests, the following parameters were measured using the 7180 automatic analyzer (Hitachi High-Technologies Corporation): aspartate aminotransferase (AST), alanine aminotransferase (ALT), alkaline phosphatase (ALP), lactate dehydrogenase (LD), creatine kinase (CK), glucose (GLU), total bilirubin (BIL), urea nitrogen (UN), creatinine (CK), total cholesterol (CHO), triglycerides (TG), phospholipids (PL), inorganic phosphorus (IP), calcium (CA), sodium (NA), potassium (K), chloride (CL), total protein (TP), albumin (ALB), and albumin/globulin ratio (A/G).

(5) Results

As a result of examining toxicological changes following a single intravenous administration of humanized anti-human HVEM antibody (HVs80) at 100 mg/kg or 300 mg/kg to groups of two male cynomolgus monkeys respectively, no influence attributable to the test substance was observed in any animal with respect to general condition, body weight, food consumption, hematological tests, or blood biochemical tests.

Example 12: Effects of humanized anti-human HVEM antibody on proliferation of Mesenchymal subtype cells

(1) Cell proliferation assay

Mesenchymal subtype GBM cells (U3054MG) were seeded at a density of 1 x $10^3$ cells/well and treated with humanized anti-human HVEM antibody (HVR81) or anti-GFP antibody (negative control) at concentrations ranging from 1 nM to 1 μM. The number of viable cells was quantified by a CCK-8 assay at the time point 7 days after treatment and normalized with the quantitative value under the antibody untreated condition to obtain a dose-response curve.

(2) Results

The results were as shown in Fig. 8. It was shown that in Mesenchymal subtype GBM cells treated with the humanized anti-human HVEM antibody (HVR81), cell proliferation was suppressed as compared with the negative control.

Example 13: Effects of humanized anti-human HVEM antibody on interaction between human BTLA and human HVEM and interaction between human APRIL and human HVEM

(1) Methods

A recombinant protein of human HVEM was immobilized on an immunoassay plate, and the humanized anti-human HVEM antibody (HVR81) or an anti-GFP antibody (negative control) was reacted for 1 hour. After washing, recombinant protein of human BTLA-His or Hemagglutinin (HA)-human APRIL was reacted for 1 hour. After washing, the HRP-labeled anti-His antibody or anti-HA antibody was reacted for 30 minutes. After further washing, 100 $\mu$L of a TMB substrate solution was added thereto for reaction for 5 minutes. 50 $\mu$L of 2 N sulfuric acid was added to stop the enzymatic reaction. The absorbance at 450 nm was measured with a plate reader, and the amount of the human BTLA-His recombinant protein or the HA-human APRIL recombinant protein binding to the human HVEM recombinant protein was quantified. In each test group, the binding amount of HVEM to BTLA or APRIL in the presence of the antibody was evaluated, and the $IC_{50}$ was calculated from the sigmoidal curve.

(2) Results

The results were as shown in Figs. 9 and 10. The $IC_{50}$ of the humanized anti-human HVEM antibody (HVR81) for BTLA-HVEM was 0.722 nM, and the $IC_{50}$ of the humanized anti-human HVEM antibody (HVR81) for APRIL-HVEM was 19.5 nM. These results demonstrated that the humanized anti-human HVEM antibody (HVR81) inhibits the binding between human BTLA and human HVEM and the binding between human APRIL and human HVEM in a concentration-dependent manner. That is, it was shown that the humanized anti-human HVEM antibody (HVR81) has an anti-glioma effect through inhibition of HVEM and has an antitumor immunity inducing effect through inhibition of BTLA.

Example 14: Suppression of NF-$\kappa$B signal via HVEM by humanized anti-human HVEM antibody

(1) Reporter assay of NF-$\kappa$B signal

The pCS-NF-$\kappa$B-RE-minP-Luc2-CMV-hRluc plasmid was constructed by cloning the firefly luciferase gene (Luc2, Promega) downstream of a minimal promoter comprising the NF-$\kappa$B response sequence, and the Renilla luciferase gene (hRluc, Promega) downstream of the MV promoter. pCS-NF-$\kappa$B-RE-minP-Luc2-CMV-hRluc, pCAG-HIVgp, and pCMV-RSV-G-Rev were transfected into HEK293FT cells using Lipofectamine 2000 Reagent (Thermo Fisher Scientific) to produce lentivirus. The culture supernatant comprising lentiviral particles was collected and concentrated using a Lenti-X Concentrator (TAKARA Bio). The concentrated lentivirus was suspended in serum-free medium and used to infect U3054MG cells, a Mesenchymal subtype GBM cell line, thereby introducing the NF-$\kappa$B-responsive Luc2 gene and the constitutively expressed hRluc gene. Mesenchymal subtype GBM cells were seeded at a density of 1 x $10^3$ cells/well and treated with humanized anti-human HVEM antibody (HVR81) or anti-GFP antibody (negative control) at concentrations ranging from 1 pM to 1 $\mu$M. At 48 hours after treatment, NF-$\kappa$B activity was evaluated using the Dual luciferase reporter assay kit (Promega). Within the same well, the luminescence of Luc2 was corrected by the luminescence of hRluc, and then normalized to the quantitative value under the antibody untreated condition to generate a dose-response curve. In addition, relative NF-$\kappa$B activity in the presence of the antibody was evaluated, and the $IC_{50}$ was calculated from the sigmoidal curve.

(2) Results

The results were as shown in Fig. 11. In addition, the $IC_{50}$ of the humanized anti-human HVEM antibody (HVR81) for relative NF-$\kappa$B activity was 14.2 nM. These results demonstrated that in Mesenchymal subtype GBM cells treated with the humanized anti-human HVEM antibody (HVR81), the activity of NF-$\kappa$B was suppressed as compared with the negative control.

Example 15: Lysosomal uptake test of humanized anti-human HVEM antibody (HVR80) in HVEM expressing cells

(1) Test methods

In order to visualize the localization of the humanized anti-human HVEM antibody (HVR80) taken up into lysosomes within HVEM-expressing cells, the humanized anti-human HVEM antibody (HVR80) and mouse IgG (negative control) were fluorescently labeled using AcidiFluor ORANGE-NHS (Goryo Chemical, Inc.), which emits strong fluorescence in acidic environments such as in lysosomes. Each antibody at the final concentration of 10 μg/ml was added to the culture medium in which HEK293T cells expressing HVEM (HEK293T-HVEM) had been cultured. In addition, only the labeled humanized anti-human HVEM antibody (HVR80) was added to the culture medium in which HeLa cells having negligible HVEM expression (negative control) were proliferated. All the cells were cultured in Dulbecco's Modified Eagles Medium (DMEM) comprising 10% fetal bovine serum (Thermo Fisher Scientific) in which complements were inactivated in advance. At 20 hours after the addition of each antibody to the cell culture solution, each cell was observed using a confocal microscope (Zeiss) (excitation wavelength: 544 nm, emission wavelength: 565 nm).

(2) Results

The results were as shown in Fig. 12. Fluorescence (black regions) was observed only when the humanized anti-human HVEM antibody (HVR80) was administered to HEK293T cells expressing HVEM, indicating the lysosomal uptake of the humanized anti-human HVEM antibody (HVR80).

Example 16: Effects of humanized anti-human HVEM antibody (HVR80) conjugated with MMAE or PNU on HEK293T-HVEM cells

(1) Methods

HEK293T cells stably overexpressing HVEM (HEK293T-HVEM) and wild-type HEK293T cells as a negative control were cultured in Dulbecco's Modified Eagle Medium (DMEM) supplemented with 10% heat-inactivated fetal bovine serum (Thermo Fisher Scientific) at 37°C in a 5% $CO_2$ atmosphere. Each cell line was seeded into each well of a 96-well plate at a density of $1 \times 10^4$ cells per well and cultured for 24 hours. After that, cells were treated with humanized anti-human HVEM antibody (HVR80) conjugated with monomethyl auristatin E (MMAE) using Glyclick ADC MMAE (Genovis AB), the humanized anti-human HVEM antibody (HVR80) conjugated with PNU using Glyclick ADC PNU (Genovis AB), or, as a negative control, the humanized anti-human HVEM antibody (HVR80), each at an antibody concentration ranging from 0.001 to 10 μg/mL. At 3 days after administration, cell viability (%) in each test group was evaluated using the Cell Count Reagent SF (Nacalai Tesque Inc.), and the $IC_{50}$ was calculated from the sigmoidal curve representing cell viability.

(2) Results

The results of cell viability were as shown in Figs. 13 and 14. In addition, the $IC_{50}$ of the humanized anti-human HVEM antibody (HVR80) conjugated with MMAE was 70 ng/ml (0.9 nM), and the $IC_{50}$ of the humanized anti-human HVEM antibody (HVR80) conjugated with PNU was 30 ng/ml (0.4 nM). From the above, it was demonstrated that the antibody-drug conjugate (ADC) of the humanized anti-human HVEM antibody (HVR80) exhibits a strong proliferation-inhibitory effect on HVEM-overexpressing HEK293T cells.

**Claims**

1.  A human HVEM-binding protein comprising:

    at least one immunoglobulin single variable region, wherein
    the immunoglobulin single variable region includes complementarity determining regions 1 to 3 (CDR1, CDR2, and CDR3), each having an amino acid sequence represented by SEQ ID NO: 1, an amino acid sequence represented by SEQ ID NO: 2, and an amino acid sequence represented by SEQ ID NO: 3, respectively, and
    the immunoglobulin single variable region is a humanized VHH.

2.  The HVEM-binding protein according to claim 1, wherein

    the immunoglobulin single variable region includes an amino acid sequence of (v), (vi), or (vii) below,
    (v) an amino acid sequence of SEQ ID NO: 18, 19, or 20,
    (vi) an amino acid sequence having at least 80% identity to the sequence of the framework region (excluding amino acids corresponding to a humanized region) in the sequence of (v),

(vii) an amino acid sequence in which one or more amino acids are deleted, substituted, inserted, and/or added in the sequence of the framework region (excluding amino acids corresponding to the humanized region) in the sequence described in (v) or (vi).

3. The HVEM-binding protein according to claim 1,
   wherein
   the immunoglobulin single variable region includes framework regions (FR1, FR2, FR3, and FR4), and the amino acid sequences of FR1, FR2, FR3, and FR4 are (i), (ii), or (iii) below,

   (i) FR1 having an amino acid sequence represented by SEQ ID NO: 5,

   FR2 having an amino acid sequence represented by SEQ ID NO: 8,
   FR3 having an amino acid sequence represented by SEQ ID NO: 11, and
   FR4 having an amino acid sequence represented by SEQ ID NO: 13,

   (ii) FR1 having an amino acid sequence represented by SEQ ID NO: 5,

   FR2 having an amino acid sequence represented by SEQ ID NO: 7,
   FR3 having an amino acid sequence represented by SEQ ID NO: 10, and
   FR4 having an amino acid sequence represented by SEQ ID NO: 13, and

   (iii) FR1 having an amino acid sequence represented by SEQ ID NO: 5,

   FR2 having an amino acid sequence represented by SEQ ID NO: 6,
   FR3 having an amino acid sequence represented by SEQ ID NO: 10, and
   FR4 having an amino acid sequence represented by SEQ ID NO: 13.

4. The HVEM-binding protein according to claim 1 or 2, further comprising a human immunoglobulin Fc region or a human serum albumin-binding protein.

5. The HVEM-binding protein according to claim 4, wherein the immunoglobulin Fc region is a human IgG4 Fc region.

6. The HVEM-binding protein according to claim 5, wherein the amino acid sequence of the human IgG4 Fc region is the amino acid sequence of SEQ ID NO: 14, 15, or 16.

7. An antibody-drug conjugate comprising the HVEM-binding protein according to claim 1 or 4 and a drug.

8. The antibody-drug conjugate according to claim 7, wherein the drug is an anticancer agent.

9. A pharmaceutical composition comprising the HVEM-binding protein according to claim 1 or 2, or the antibody-drug conjugate according to claim 7.

10. The pharmaceutical composition according to claim 9 for use in inhibiting the binding of HVEM to BTLA and/or APRIL.

11. The pharmaceutical composition according to claim 9 for use in the treatment or prevention of solid cancer.

12. The pharmaceutical composition according to claim 9 for use in the treatment or prevention of gliomas.

13. The pharmaceutical composition according to claim 12, wherein the gliomas are any one of an oligodendroglioma, an oligoastrocytoma, an astrocytoma, or a glioblastoma multiforme.

14. The pharmaceutical composition according to claim 13, wherein the glioblastoma multiforme belongs to any one of the Proneural subtype, Neural subtype, Classical subtype, or Mesenchymal subtype.

15. The pharmaceutical composition according to claim 9 for use in combination with another/other anticancer agent(s).

16. An agent for inhibiting the HVEM signaling pathway, comprising the HVEM-binding protein according to claim 1 or 2.

17. The agent according to claim 16, wherein the HVEM signaling pathway is an NF-κB signaling pathway via HVEM.

18. An agent for inducing anticancer immunity comprising the HVEM-binding protein according to claim 1 or 2.

19. A polynucleotide encoding the HVEM-binding protein according to claim 1 or 2.

20. An expression vector comprising the polynucleotide according to claim 19.

21. A method for treating or preventing solid cancer, comprising administering to a subject in need thereof the HVEM-binding protein according to claim 1 or 2 or a composition including the same, or the antibody-drug conjugate according to claim 7 or a composition including the same.

22. Use of the HVEM-binding protein according to claim 1 or 2, or the antibody-drug conjugate according to claim 7, for the manufacture of a medicament for treating or preventing solid cancer.

**FIGURE 1**

(a)

(b)

**FIGURE 2**

FIGURE 3

FIGURE 4

FIGURE 5

FIGURE 6

FIGURE 7

**FIGURE 8**

**FIGURE 9**

EP 4 759 829 A1

**FIGURE 10**

**FIGURE 11**

36

HEK293T-HVEM treated with mouse IgG    HeLa cells treated with HVR80    HEK293T-HVEM treated with HVR80

**FIGURE 12**

-○- HEK293T (wild-type) treated with HVR80

-●- HEK293T-HVEM treated with HVR80

-□- HEK293T (wild-type) treated with MMAE-conjugated HVR80

-■- HEK293T-HVEM treated with MMAE-conjugated HVR80

**FIGURE 13**

-○- HEK293T (wild-type) treated with HVR80

-●- HEK293T-HVEM treated with HVR80

-□- HEK293T (wild-type) treated with PNU-conjugated HVR80

-■- HEK293T-HVEM treated with PNU-conjugated HVR80

**FIGURE 14**

## INTERNATIONAL SEARCH REPORT

| International application No. |
| --- |
| **PCT/JP2024/028824** |

| A. | CLASSIFICATION OF SUBJECT MATTER |
| --- | --- |

*C07K 16/28*(2006.01)i; *A61K 39/395*(2006.01)i; *A61K 45/00*(2006.01)i; *A61K 47/68*(2017.01)i; *A61P 35/00*(2006.01)i; *A61P 43/00*(2006.01)i; *C07K 16/46*(2006.01)i; *C12N 15/13*(2006.01)i; *C12N 15/63*(2006.01)i

FI:   C07K16/28 ZNA; C07K16/46; C12N15/63 Z; A61K47/68; A61P43/00 111; A61P35/00; A61K45/00; A61K39/395 C; C12N15/13

According to International Patent Classification (IPC) or to both national classification and IPC

| B. | FIELDS SEARCHED |
| --- | --- |

Minimum documentation searched (classification system followed by classification symbols)

C07K16/28; A61K39/395; A61K45/00; A61K47/68; A61P35/00; A61P43/00; C07K16/46; C12N15/13; C12N15/63

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 2020/138503 A1 (THE UNIVERSITY OF TOKYO) 02 July 2020 (2020-07-02) claims, table 9, paragraph [0060], examples | 1-22 |
| Y | WO 2020/004492 A1 (KYOWA KIRIN CO., LTD.) 02 January 2020 (2020-01-02) examples | 1-22 |
| Y | WO 2023/112965 A1 (TEIKYO HEISEI UNIVERSITY) 22 June 2023 (2023-06-22) examples | 1-22 |
| Y | WO 2023/006040 A1 (JIANGSU SIMCERE PHARMACEUTICAL CO., LTD.) 02 February 2023 (2023-02-02) examples | 1-22 |
| Y | WO 2023/040945 A1 (JIANGSU HENGRUI PHARMACEUTICALS CO., LTD.) 23 March 2023 (2023-03-23) examples | 4-8, 21, 22 |

☑ Further documents are listed in the continuation of Box C.    ☑ See patent family annex.

| | |
| --- | --- |
| *     Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **15 October 2024** | **22 October 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/028824**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2023-516280 A (VIB VZW) 19 April 2023 (2023-04-19)<br>examples, particularly examples 2, 18 | 4-8, 21, 22 |

## INTERNATIONAL SEARCH REPORT

| International application No. |
| --- |
| **PCT/JP2024/028824** |

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

40

## INTERNATIONAL SEARCH REPORT
### Information on patent family members

| International application No. |
| --- |
| **PCT/JP2024/028824** |

| Patent document cited in search report | | | Publication date (day/month/year) | Patent family member(s) | | | Publication date (day/month/year) |
| --- | --- | --- | --- | --- | --- | --- | --- |
| WO | 2020/138503 | A1 | 02 July 2020 | US | 2022/0105122 | A1 | |
| | | | | claims, table 9, examples, paragraph [0129] | | | |
| | | | | EP | 3936149 | A1 | |
| WO | 2020/004492 | A1 | 02 January 2020 | US | 2021/0147541 | A1 | |
| | | | | examples | | | |
| | | | | EP | 3816290 | A1 | |
| WO | 2023/112965 | A1 | 22 June 2023 | (Family: none) | | | |
| WO | 2023/006040 | A1 | 02 February 2023 | EP | 4378954 | A1 | |
| | | | | examples | | | |
| WO | 2023/040945 | A1 | 23 March 2023 | EP | 4403574 | A1 | |
| | | | | examples | | | |
| JP | 2023-516280 | A | 19 April 2023 | US | 2023/0227537 | A1 | |
| | | | | examples | | | |
| | | | | WO | 2021/156490 | A2 | |
| | | | | EP | 4100055 | A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023131057 A

- WO 2020138503 A

### Non-patent literature cited in the description

- **SAVARY K et al.** *Oncogene*, 2013, vol. 32, 5409-5420
- **DALMO et al.** *Mol. Cancer Res.*, 2020, vol. 18, 981-991
- **PASERO C et al.** *Curr Opin Pharmacol.*, 2012, vol. 12, 478-485
- **SEDY JR** ; **RAMEZANI-RAD P**. *Adv. Cancer Res.*, 2019, vol. 42, 145-186
- **KABAT et al.** Sequences of Proteins of Immunological Interest. 1991
- **CHOTHIA** ; **LESK**. *J. Mol. Biol.*, 1987, vol. 196, 901-917
- **WILBUR**. *Natl. Acad. Sci. U.S.A.*, 1983, vol. 80, 726-730
- **ALTSCHUL et al.** *J. Mol. Biol.*, 1990, vol. 215, 403-410
- **PEASRON et al.** *Methods in Enzymology*, 1990, vol. 183, 63-69
- **SMITH-WATERMAN**. *Meth. Enzym*, 1988, vol. 164, 765
- **WATANABE et al.** *Nat. Immunol.*, 2003, vol. 4, 670-679
- **CHEN** ; **MELLMAN**. *Immunity*, 2013, vol. 39, 1-10
- **WOJCIECHOWICZ et al.** *Eur. J. Med. Chem.*, 2024, vol. 268, 116231
- **SORDO-BAHAMONDE et al.** *Mol. Cancer*, 2023, vol. 22, 142
- **NOWACKA** ; **JABLONSKA**. *Cytokine Growth Factor Rev.,* 2021, vol. 61, 38-44
- **YAMINI**. *Cells*, 2018, vol. 7, 125
- **BHAT et al.** *Cancer Cell*, 2013, vol. 24, 331-346
- **VERHAAK R G et al.** *Cancer Cell*, 2010, vol. 17, 98-110
- General Rules for Preparations of the Japanese Pharmacopoeia
- **SWINDELLS et al.** *J Mol Biol.*, 2017, vol. 429, 356-364, http://www.abysis.org/abysis/index.html